(19) Europäisches Patentamt
European Patent Office
Office européen des brevets

(11) EP 4 468 440 B1

# (12) EUROPEAN PATENT SPECIFICATION

(45) Date of publication and mention of the grant of the patent:
**06.05.2026 Bulletin 2026/19**

(21) Application number: **22921668.4**

(22) Date of filing: **07.12.2022**

(51) International Patent Classification (IPC):
***H01M 10/0567*** *(2010.01)* ***H01M 10/0569*** *(2010.01)*
***H01M 10/0525*** *(2010.01)* ***H01M 10/052*** *(2010.01)*
***H01M 10/0568*** *(2010.01)* ***H01M 10/42*** *(2006.01)*

(52) Cooperative Patent Classification (CPC):
**H01M 10/0567; H01M 10/052; H01M 10/0525; H01M 10/0568; H01M 10/0569; H01M 10/4235;**
H01M 2300/0025; Y02E 60/10

(86) International application number:
**PCT/CN2022/137107**

(87) International publication number:
**WO 2023/138244 (27.07.2023 Gazette 2023/30)**

## (54) NON-AQUEOUS ELECTROLYTE SOLUTION AND SECONDARY BATTERY

NICHTWÄSSRIGE ELEKTROLYTLÖSUNG UND SEKUNDÄRBATTERIE

SOLUTION D'ÉLECTROLYTE NON AQUEUX ET BATTERIE SECONDAIRE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **21.01.2022 CN 202210073247**

(43) Date of publication of application:
**27.11.2024 Bulletin 2024/48**

(73) Proprietor: **Shenzhen Capchem Technology Co., Ltd**
**Shenzhen, Guangdong 518118 (CN)**

(72) Inventors:
- **QIAN, Yunxian**
  **Shenzhen, Guangdong 518118 (CN)**
- **LIU, Zhongbo**
  **Shenzhen, Guangdong 518118 (CN)**
- **HU, Shiguang**
  **Shenzhen, Guangdong 518118 (CN)**
- **DENG, Yonghong**
  **Shenzhen, Guangdong 518118 (CN)**
- **WANG, Yong**
  **Shenzhen, Guangdong 518118 (CN)**
- **HUANG, Xiong**
  **Shenzhen, Guangdong 518118 (CN)**

(74) Representative: **Petraz, Gilberto Luigi et al**
**GLP S.r.l.**
**Viale Europa Unita, 171**
**33100 Udine (IT)**

(56) References cited:
**WO-A1-2021/135900**
**CN-A- 108 242 568**
**CN-A- 110 931 863**
**CN-A- 111 755 753**
**CN-A- 112 349 961**
**CN-A- 112 349 961**
**CN-A- 114 552 004**
**CN-A- 115 117 446**
**JP-A- 2015 149 234**
**JP-A- 2015 162 289**
**JP-A- 2015 187 926**
**KR-A- 20180 136 655**
**US-A1- 2021 296 703**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 468 440 B1

## Description

### FIELD

The present invention relates to the technical field of energy storage battery devices, and more particularly to a non-aqueous electrolyte and a secondary battery.

### BACKGROUND

A lithium-ion battery has been widely used in the field of 3C digital products such as mobile phones and notebook computers, as well as in the field of new energy vehicles due to its advantages of high operating voltage, wide operating temperature range, high energy density and power density, no memory effect and long cycle life. In recent years, with a continuous development of light and thin 3C digital products, a demand for a high energy density of the lithium-ion battery in a battery industry is higher and higher. At the same time, for a user side, fast charging has become a basic requirement of a battery. Therefore, there is an urgent need to increase an energy density of lithium-ion battery and improve a fast charging performance. CN112349961A discloses a non-aqueous electrolyte, comprising LiPF6, a mixture of ethylene carbonate (EC), propylene carbonate (PC) diethyl carbonate (DEC) and an additive.

At present, there are mainly two ways to increase an energy density of a battery. One way is to increase a cutoff charge voltage of a positive electrode, and the other way is to pressurize an electrode active material layer to achieve a high density. However, after the cutoff charge voltage of the positive electrode is increased, the activity of the positive electrode is further improved. A side reaction between the positive electrode and an electrolyte is also exacerbated, resulting in the dissolution of transition metal ions at the positive electrode, thus resulting in the deterioration of a cycle performance of the battery. In addition, by using a high compaction electrode, a load of an electrode sheet may be improved, thus improving an overall energy density of the battery. However, due to a low porosity of the high compaction electrode, a liquid retention capacity of the battery is also reduced, so that the electrolyte is difficult to penetrate at an interface of the electrode sheet with a low porosity, thus increasing a contact internal resistance between the electrolyte and the electrode. The polarization of charge and discharge becomes larger during long term cycling, resulting in a sudden dive due to lithium precipitation. A lithium ion conduction channel of a high compaction electrode sheet is tortuous, resulting in difficulty in lithium ion transmission, so that a fast charging performance of the battery is very poor. To sum up, the way of increasing the energy density in the existing technology leads to the deterioration of the cycle performance and fast charging performance of the battery. Therefore, how to enable a high-voltage and high compaction lithium-ion battery to have good fast charging performance is an industry problem, which needs to be improved from various aspects such as an electrode material and an electrolyte.

From the perspective of electrolyte, in the prior art, a carboxylate system with a high dielectric constant and a small viscosity is often selected as a solvent to improve the fast charging performance of the battery. However, the carboxylate is unstable under a high voltage and tends to generate decomposition products at a positive electrode side. The decomposition products migrate to a negative electrode to be reduced, and accumulate on a surface of the negative electrode, resulting in the increase of an impedance of the battery and rapid deterioration in later stages of the cycle. Therefore, it is urgent to improve the fast charging performance of the battery from the perspective of electrolyte.

### SUMMARY

In view of a problem that it is difficult to combine an energy density, a cycle performance and a fast charging performance of an existing secondary battery, the present invention provides a non-aqueous electrolyte and a secondary battery.

The technical solution used in the present invention to solve the above-mentioned technical problem is as follows.

On the one hand, the present invention provides a non-aqueous electrolyte. The non-aqueous electrolyte includes an electrolyte salt, a non-aqueous organic solvent, and an additive. The non-aqueous organic solvent includes a carboxylate, and the additive includes a compound represented by formula 1:

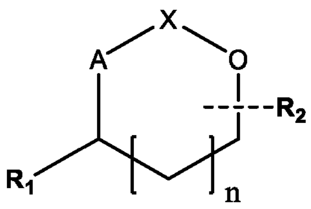

Formula 1

where n is 0 or 1, A is selected from C or O, X is selected from

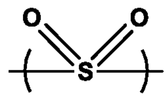

or

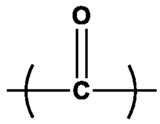

,

$R_1$ and $R_2$ are each independently selected from H,

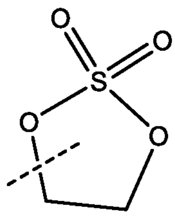

,

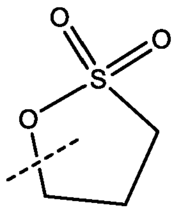

,

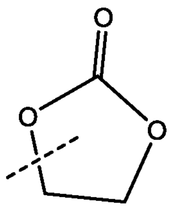

,

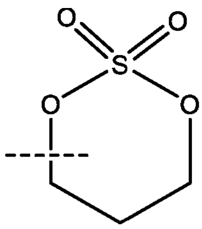

, or

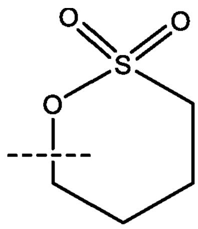

;

$R_1$ and $R_2$ are not selected from H simultaneously, and X, $R_1$ and $R_2$ at least contain one sulfur atom.

The non-aqueous electrolyte satisfies following conditions: $0.02 \le an/m \le 9$, $0.01\% \le a \le 5\%$, $5\% \le m \le 70\%$, and $8\% \le n \le 25\%$;

where a is a mass percentage content of the compound represented by the formula 1 in the non-aqueous electrolyte, in %;

m is a mass percentage content of the carboxylate in the non-aqueous electrolyte, in %; and

n is a mass percentage content of the electrolyte salt in the non-aqueous electrolyte, in %.

A conductivity of the non-aqueous electrolyte at 25°C is greater than or equal to 5mS/cm.

Optionally, the non-aqueous electrolyte satisfies a following condition: $0.2 \le an/m \le 4$.

Optionally, the mass percentage content a of the compound represented by the formula 1 in the non-aqueous electrolyte is 0.05% to 3%.

Optionally, the mass percentage content m of the carboxylate in the non-aqueous electrolyte is 10% to 60%.

Optionally, the mass percentage content n of the electrolyte salt in the non-aqueous electrolyte is 10% to 20%.

Preferably, the electrolyte salt is selected from one or more of: $LiPF_6$, $LiPO_2F_2$, $LiBF_4$, $LiClO_4$, $LiCF_3SO_3$,

$LiN(SO_2CF_3)_2$ and $LiN(SO_2F)_2$.

Optionally, the compound represented by the formula 1 is selected from one or more of following compounds 1-22:

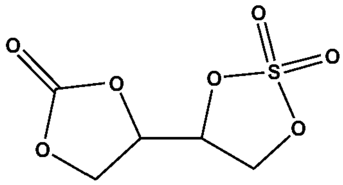

Compound 1

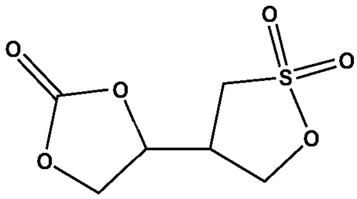

Compound 2

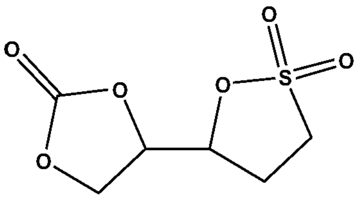

Compound 3

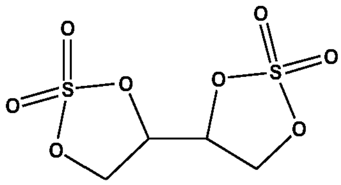

Compound 4

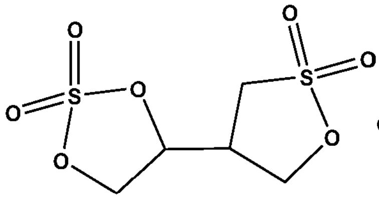

Compound 5

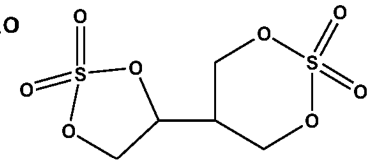

Compound 6

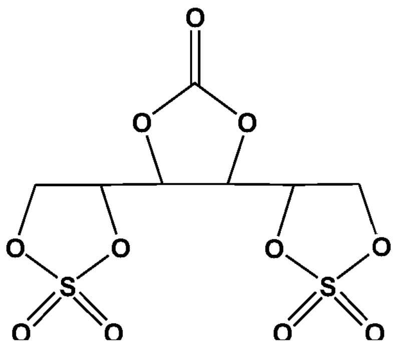

Compound 7

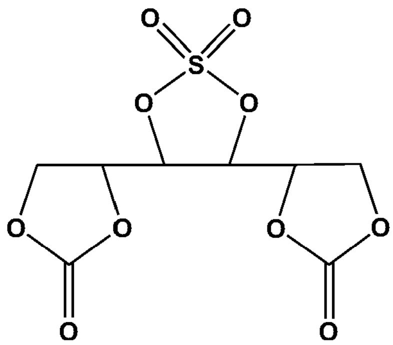

Compound 8

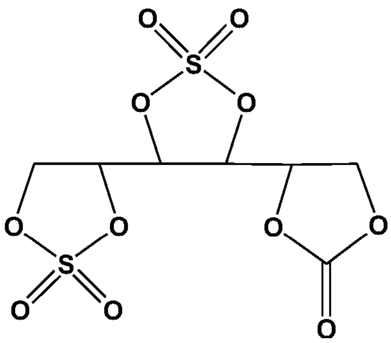

Compound 9

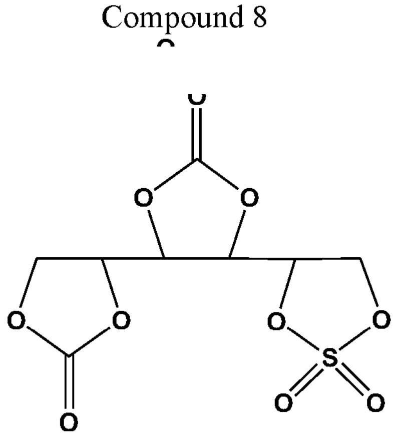

Compound 10

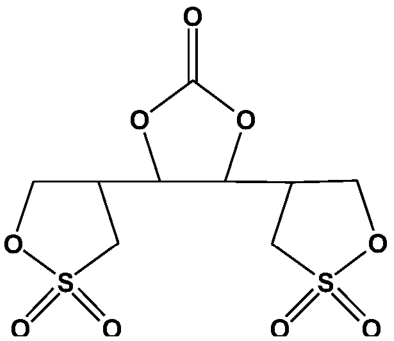

Compound 11

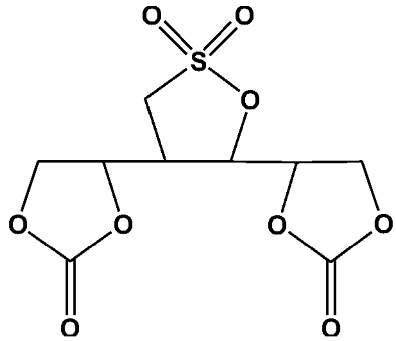

Compound 12

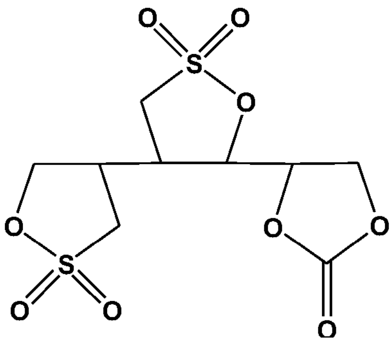

Compound 13

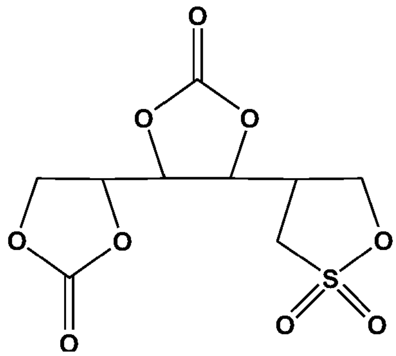

Compound 14

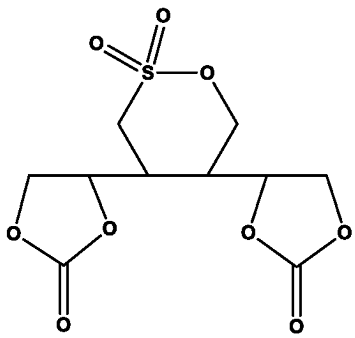

Compound 15

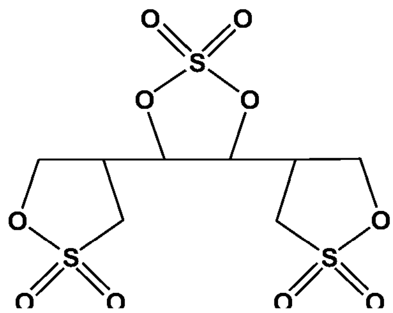

Compound 16

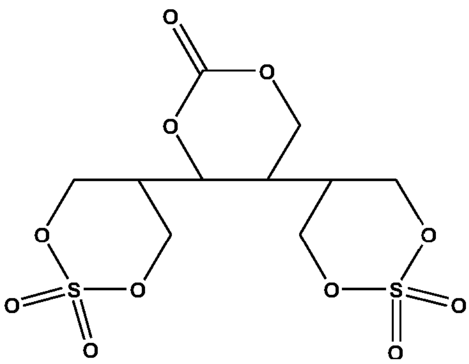

Compound 17

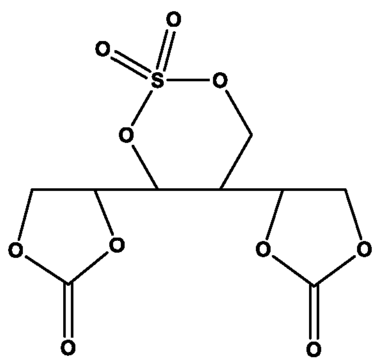

Compound 18

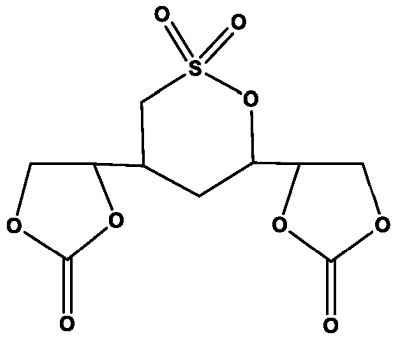

Compound 19

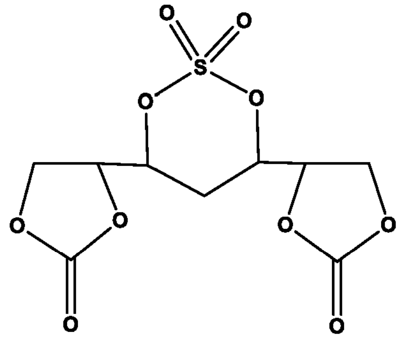

Compound 20

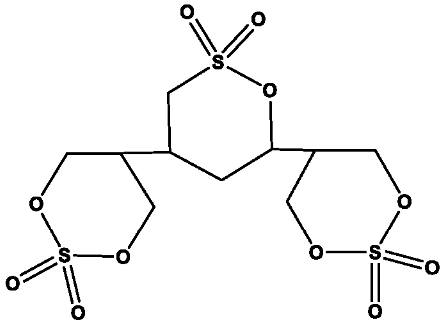

Compound 21

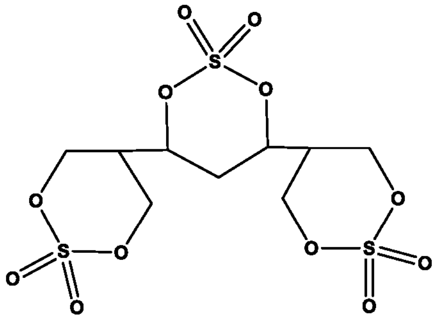

Compound 22

Optionally, the carboxylate includes one or more of: methyl acetate, ethyl acetate, propyl acetate, methyl propionate, ethyl propionate, propyl propionate, 2,2-difluoroethyl acetate, methyl butyrate, methyl isobutyrate, methyl trimethylacetate and ethyl trimethylacetate.

Optionally, the non-aqueous electrolyte further includes an auxiliary additive. The auxiliary additive includes at least one of: a cyclic sulfate compound, a sultone compound, a cyclic carbonate compound, an unsaturated phosphate compound, a nitrile compound and a lithium oxalate borate.

Preferably, the auxiliary additive is added in an amount of 0.01% to 30% based on a total mass of the non-aqueous electrolyte as 100%.

Preferably, the cyclic sulfate compound is selected from at least one of: ethylene sulfate, propene sulfate or methyl ethylene sulfate. The sultone compound is selected from at least one of: 1,3-propane sultone, 1,4-butane sultone or 1,3-propene sultone. The cyclic carbonate compound is selected from at least one of: vinylene carbonate, vinyl ethylene carbonate, fluoroethylene carbonate or a compound represented by formula 2

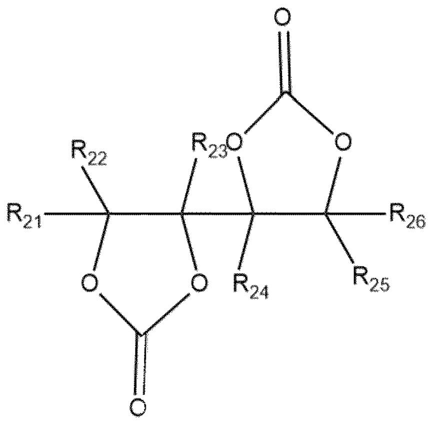

Formula 2

in the formula 2, $R_{21}$, $R_{22}$, $R_{23}$, $R_{24}$, $R_{25}$ and $R_{26}$ are each independently selected from one of: a hydrogen atom, a halogen

atom or a C1-C5 group.

The unsaturated phosphate compound is selected from at least one of compounds represented by formula 3:

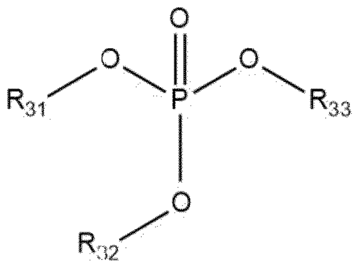

Formula 3

in the formula 3, $R_{31}$, $R_{32}$ and $R_{33}$ are each independently selected from a C1-C5 saturated hydrocarbon group, an unsaturated hydrocarbon group, a halogenated hydrocarbon group and $-Si(C_mH_{2m+1})_3$, where m is a natural number of 1 to 3, and at least one of $R_{31}$, $R_{32}$ and $R_{33}$ is an unsaturated hydrocarbon group.

The nitrile compound is selected from one or more of: succinonitrile, glutaronitrile, ethylene glycol bis(propionitrile)ether, hexane trinitrile, adiponitrile, pimelonitrile, suberonitrile, azelanitrile and sebaconitrile.

The lithium oxalate borate is selected from one or more of: $Li[B(C_2O_4)_2]$ and $Li[B(C_2O_4)F_2]$.

Preferably, the non-aqueous organic solvent further includes one or more of: cyclic carbonates, linear carbonates and ethers.

Preferably, the cyclic carbonates include one or more of: vinylene carbonate, propene carbonate and ethylene carbonate. The linear carbonates include one or more of: dimethyl carbonate, diethyl carbonate and ethyl methyl carbonate. The ethers include one or more of: glycol dimethyl ether, 1,3-dioxolane and 1,1,2,2-tetrafluoroethyl-2,2,3,3-tetrafluoropropyl ether.

On the other hand, the present invention provides a secondary battery. The secondary battery includes a positive electrode, a negative electrode, and a non-aqueous electrolyte as described above. The positive electrode includes a positive electrode material layer, and a compaction density of the positive electrode material layer is $3.5g/cm^3$ or more. The negative electrode includes a negative electrode material layer, and a compaction density of the negative electrode material layer is $1.5g/cm^3$ or more. Therefore, a battery with a high energy density may be obtained.

According to the non-aqueous electrolyte provided in the present invention, aiming at characteristics of a high compaction density and a high voltage in a high energy density battery system, the carboxylate with a high dielectric constant and a small viscosity is selected as the solvent of the non-aqueous electrolyte, so as to improve infiltration and ion conductivity of the non-aqueous electrolyte to the positive and negative electrodes, and improve the fast charging ability of the battery. Aiming at a problem that the carboxylate is unstable and tends to decompose under a high voltage, the inventors have found through a large number of researches that a side reaction of the carboxylate in the battery may be effectively inhibited by adding the compound represented by the formula 1 as the additive into the non-aqueous electrolyte. The content of electrolyte salt and the content of carboxylate jointly affect the ion conductivity of the non-aqueous electrolyte. By controlling parameter ranges of the mass percentage content a of the compound represented by the formula 1 in the non-aqueous electrolyte, the mass percentage content m of the carboxylate in the non-aqueous electrolyte and the mass percentage content n of the electrolyte salt in the non-aqueous electrolyte, and allowing them to satisfy a relational expression of $0.02 \leq an/m \leq 9$, a stable and dense passivation film may be formed on the positive and negative electrodes, and the oxidation of the carboxylate is effectively prevented. At the same time, an internal resistance of the battery is effectively reduced and the fast charging performance and the cycle performance of the battery are improved by limiting a content relation of the compound as represented by formula 1, the carboxylate and the electrolyte salt.

## DETAILED DESCRIPTION

In order that the technical problems, technical solutions and advantageous effects to be solved by the present invention may be more clearly understood, the present invention will be described in further detail with reference to embodiments. It is to be understood that particular embodiments described herein are only used to explain the present invention, and are not intended to limit the present invention.

An embodiment of the present invention provides a non-aqueous electrolyte. The non-aqueous electrolyte includes an electrolyte salt, a non-aqueous organic solvent, and an additive. The non-aqueous organic solvent includes a carboxylate, and the additive includes a compound represented by formula 1:

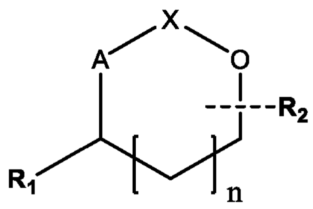

Formula 1

where n is 0 or 1, A is selected from C or O, X is selected from

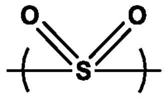

or

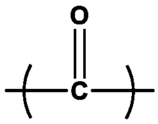,

$R_1$ and $R_2$ are each independently selected from H,

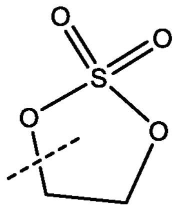,

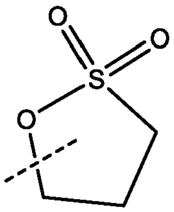, 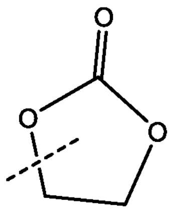, 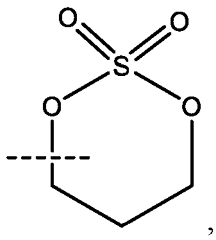, or 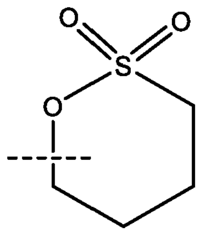,

$R_1$ and $R_2$ are not selected from H simultaneously, and X, $R_1$ and $R_2$ at least contain one sulfur atom.

The non-aqueous electrolyte satisfies following conditions: $0.02 \leq an/m \leq 9$, $0.01\% \leq a \leq 5\%$, $5\% \leq m \leq 70\%$, and $8\% \leq n \leq 25\%$;

where a is a mass percentage content of the compound represented by the formula 1 in the non-aqueous electrolyte, in %;
m is a mass percentage content of the carboxylate in the non-aqueous electrolyte, in %; and
n is a mass percentage content of the electrolyte salt in the non-aqueous electrolyte, in %.

A conductivity of the non-aqueous electrolyte at 25°C is greater than or equal to 5mS/cm.

The carboxylate is unstable under a high voltage and tends to produce decomposition products at a positive electrode side. Some of the decomposition products migrate to the negative electrode to be reduced, and accumulate on a surface of the negative electrode, resulting in a continuous increase in an impedance of the battery, which is not conducive to the improvement of a cycle performance of the battery. Through a large number of researches, the inventors have found that a side reaction of the carboxylate in the battery may be effectively inhibited by adding the compound represented by the formula 1 as the additive into the non-aqueous electrolyte. It is speculated that due to the fact that the compound

represented by the formula 1 participates in film formation on the positive and the negative electrodes simultaneously, a formed passivation film may effectively inhibit a decomposition reaction of the carboxylate on a surface of the positive electrode, reduce the formation of oxidation by-products, and inhibit the reduction and accumulation of the oxidation by-products on a surface of the negative electrode simultaneously. Therefore, the continuous increase of an impedance of the passivation film on the surfaces of the positive and negative electrodes may be avoided during cycling, and at the same time, the improvement effect of the carboxylate on a fast charging performance of the battery is ensured. The content of electrolyte salt and the content of carboxylate jointly affect viscosity and ion conductivity of the non-aqueous electrolyte. By controlling parameter ranges of the mass percentage content a of the compound represented by the formula 1 in the non-aqueous electrolyte, the mass percentage content m of the carboxylate in the non-aqueous electrolyte and the mass percentage content n of the electrolyte salt in the non-aqueous electrolyte, and allowing them to satisfy a relational expression of $0.02 \leq an/m \leq 9$, a stable and dense passivation film may be formed on the positive and negative electrodes, and the oxidation of the carboxylate is effectively prevented. At the same time, an internal resistance of the battery is effectively reduced and a fast charging performance and the cycle performance of the battery are improved by limiting a content relation of the compound as represented by formula 1, the carboxylate and the electrolyte salt.

The conductivity of the non-aqueous electrolyte at 25°C is greater than or equal to 5mS/cm, so that a film-forming effect of the compound represented by the formula 1 is better, and it is ensured that the electrolyte has a better high-temperature performance.

In some preferred embodiments, the conductivity of the non-aqueous electrolyte at 25°C is 6.0mS/cm to 9.0mS/cm, which may further ensure that the negative electrode does not precipitate lithium, thus improving a high-temperature cycle performance of the battery. The higher the conductivity of the electrolyte at room temperature of 25°C, the better the performance of the battery. However, in case that a content of a certain type of organic solvent is increased simply to improve the conductivity of the electrolyte at room temperature, a side reaction may be increased due to an excessive addition ratio of the certain type of organic solvent, and on the contrary, the performance such as a cycle life of the battery is affected to a certain extent.

In some embodiments, when n is 0, the compound represented by the formula 1 is:

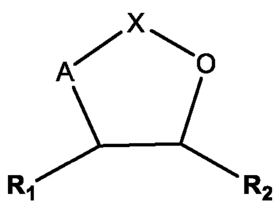;

where Ais selected from C or O, X is selected from

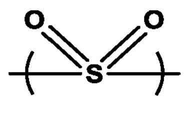 or 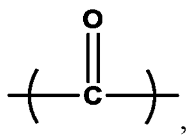,

$R_1$ and $R_2$ are each independently selected from H,

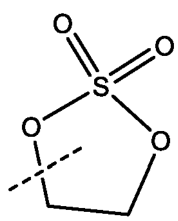, 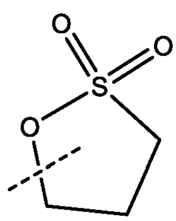,

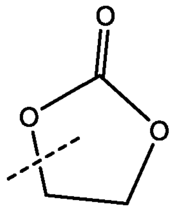 , 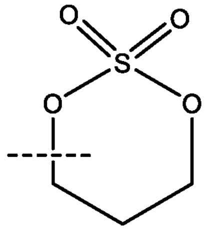 , or 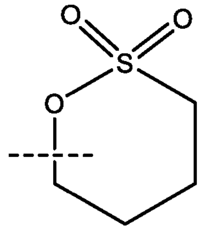 ,

$R_1$ and $R_2$ are not selected from H simultaneously, and X, $R_1$ and $R_2$ at least contain one sulfur atom.

In some embodiments, when n is 1, the compound represented by the formula 1 is:

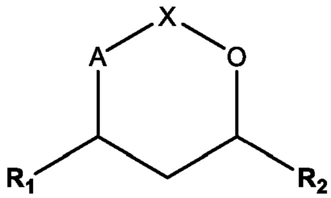 or 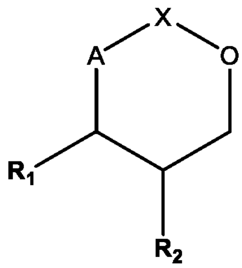 ;

where Ais selected from C or O, X is selected from

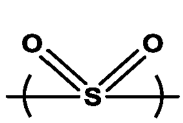 or 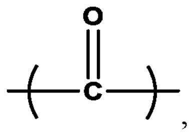 ,

$R_1$ and $R_2$ are each independently selected from H,

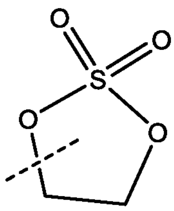 , 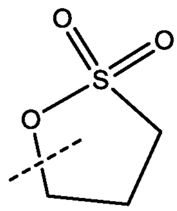 ,

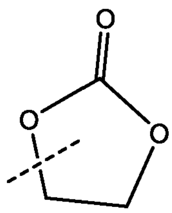 , 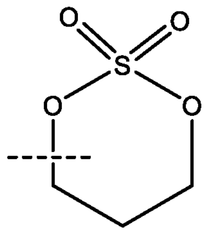 , or 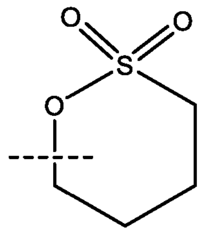 ,

$R_1$ and $R_2$ are not selected from H simultaneously, and X, $R_1$ and $R_2$ at least contain one sulfur atom.

In preferred embodiments, the non-aqueous electrolyte satisfies a following condition: $0.2 \leq an/m \leq 4$.

The mass percentage content a of the compound represented by the formula 1 in the non-aqueous electrolyte, the mass percentage content m of the carboxylate in the non-aqueous electrolyte and the mass percentage content n of the electrolyte salt in the non-aqueous electrolyte are associated, so that comprehensive effects of the compound represented by the formula 1, the carboxylate and the electrolyte salt on the fast charging performance of the battery may be integrated, the non-aqueous electrolyte suitable for a high compaction battery may be obtained, and the cycle performance of the battery under high-rate charge and discharge conditions may be improved.

In a specific embodiment, the mass percentage content a of the compound represented by the formula 1 in the non-aqueous electrolyte may be 0.01%, 0.05%, 0.1%, 0.12%, 0.15%, 0.3%, 0.5%, 0.8%, 0.9%, 1.0%, 1.2%, 1.4%, 1.7%,

1.9%, 2.1%, 2.2%, 2.4%, 2.7%, 2.9%, 3.1%, 3.3%, 3.5%, 3.7%, 4.2%, 4.4%, 4.7%, 4.9%, or 5.0%.

In preferred embodiments, the mass percentage content a of the compound represented by the formula 1 in the non-aqueous electrolyte is 0.05% to 3%.

In case that the content of the compound represented by the formula 1 in the non-aqueous electrolyte is too low, a formation quality of the passivation film on the surfaces of the positive and negative electrodes is affected, and it is difficult to effectively inhibit the side reaction of the carboxylate. In case that the content of the compound represented by the formula 1 in the non-aqueous electrolyte is too high, the viscosity of the non-aqueous electrolyte is increased, which affects the infiltration of the non-aqueous electrolyte to positive and negative electrode materials, resulting in an increase in an impedance and affecting the performance of the battery.

In specific embodiments, the mass percentage content m of the carboxylate in the non-aqueous electrolyte may be 5%, 8%, 10%, 13%, 16%, 17%, 19%, 21%, 22%, 24%, 27%, 29%, 31%, 33%, 35%, 37%, 42%, 44%, 47%, 49%, 50%, 53%, 55%, 58%, 60%, 61%, 63%, 65%, 68%, or 70%.

In preferred embodiments, the mass percentage content m of the carboxylate in the non-aqueous electrolyte is 10% to 60%.

By adding the carboxylate as the non-aqueous organic solvent into the non-aqueous electrolyte, the fast charging performance of the battery may be improved. However, in case that the carboxylate is added in a small amount, the conductivity of the non-aqueous electrolyte is low, which affects the cycle performance of the battery. In case that the carboxylate is added in an excessive amount, the stability of the non-aqueous electrolyte may be reduced, which is also not conducive to the improvement of the cycle performance of the battery.

In specific embodiments, the mass percentage content n of the electrolyte salt in the non-aqueous electrolyte may be 8%, 9%, 10%, 11%, 12%, 13%, 14%, 15%, 16%, 17%, 18%, 19%, 20%, 21%, 22%, 23%, 24%, or 25%.

In preferred embodiments, the mass percentage content n of the electrolyte salt in the non-aqueous electrolyte is 10% to 20%.

Alkali metal ions formed by the dissociation of the electrolyte salt in the non-aqueous electrolyte are deintercalated and intercalated between the positive electrode and the negative electrode to complete a cycle of charge and discharge. A concentration of the electrolyte salt directly affects a transmission speed of the alkali metal ions, and the transmission speed of the alkali metal ions affects a potential change of the negative electrode. In a fast charging process of the battery, it is necessary to increase a moving speed of the alkali metal ions as much as possible to prevent the formation of lithium dendrites caused by the rapid decrease in a potential of the negative electrode, bringing safety hazards to the battery, and prevent a cycle capacity of the battery from decaying too fast at the same time. When the content of the electrolyte salt is too low, an intercalation and deintercalation efficiency of the alkali metal ions between the positive electrode and the negative electrode may be reduced, and a demand of fast charging of the battery cannot be met. When the content of the electrolyte salt is too high, the viscosity of the non-aqueous electrolyte is increased, so that the improvement of the intercalation and deintercalation efficiency of the alkali metal ions between the positive electrode and the negative electrode is also not facilitated, and the internal resistance of the battery is increased.

In some embodiments, the electrolyte salt includes one or more of: a lithium salt, a sodium salt, a potassium salt, a magnesium salt, a zinc salt and an aluminum salt. In preferred embodiments, the electrolyte salt is selected from the lithium salt or the sodium salt.

In preferred embodiments, the electrolyte salt is selected from at least one of: $LiPF_6$, $LiPO_2F_2$, $LiBF_4$, $LiSbF_6$, $LiAsF_6$, $LiCF_3SO_3$, $LiN(SO_2CF_3)_2$, $LiC(SO_2CF_3)_3$, $LiN(SO_2C_2F_5)_2$, $LiN(SO_2F)_2$, LiCl, LiBr, LiI, $LiClO_4$, $LiBF_4$, $LiB_{10}Cl_{10}$, $LiAlCl_4$, a lithium chloroborane, a lower aliphatic lithium carboxylate having 4 or less carbon atoms, a lithium tetraphenylborate or a lithium imino group. Specifically, the electrolyte salt may be inorganic electrolyte salts such as $LiBF_4$, $LiClO_4$, $LiAlF_4$, $LiSbF_6$, $LiTaF_6$, $LiWF_7$; fluorophosphate electrolyte salts such as $LiPF_6$; tungstate electrolyte salts such as $LiWOF_5$; carboxylic acid electrolyte salts such as $HCO_2Li$, $CH_3CO_2Li$, $CH_2FCO_2Li$, $CHF_2CO_2Li$, $CF_3CO_2Li$, $CF_3CH_2CO_2Li$, $CF_3CF_2CO_2Li$, $CF_3CF_2CF_2CO_2Li$, and $CF_3CF_2CF_2CF_2CO_2Li$; sulfonic acid electrolyte salts such as $CH_3SO_3Li$; imide electrolyte salts such as $LiN(FCO_2)_2$, $LiN(FCO)(FSO_2)$, $LiN(FSO_2)_2$, $LiN(FSO_2)(CF_3SO_2)$, $LiN(CF_3SO_2)_2$, $LiN(C_2F_5SO_2)_2$, cyclic lithium 1,2-perfluoroethanedisulfonylimide, cyclic lithium 1,3-perfluoropropanedisulfonylimide, $LiN(CF_3SO_2)(C_4F_9SO_2)$; methyl electrolyte salts such as $LiC(FSO_2)_3$, $LiC(CF_3SO_2)_3$, $LiC(C_2F_5SO_2)_3$; and fluorine-containing organic electrolyte salts such as $LiPF_4(CF_3)_2$, $LiPF_4(C_2F_5)_2$, $LiPF_4(CF_3SO_2)_2$, $LiPF_4(C_2F_5SO_2)_2$, $LiBF_3CF_3$, $LiBF_3C_2F_5$, $LiBF_3C_3F_7$, $LiBF_2(CF_3)_2$, $LiBF_2(C_2F_5)_2$, $LiBF_2(CF_3SO_2)_2$, $LiBF_2(C_2F_5SO_2)_2$, etc.

In preferred embodiments, the electrolyte salt is selected from one or more of: $LiPF_6$, $LiPO_2F_2$, $LiBF_4$, $LiClO_4$, $LiCF_3SO_3$, $LiN(SO_2CF_3)_2$, and $LiN(SO_2F)_2$.

In case that the electrolyte salt is selected from other salts such as a sodium salt, a potassium salt, a magnesium salt, a zinc salt or an aluminum salt, lithium in the lithium salt may be correspondingly replaced with sodium, potassium, magnesium, zinc, or aluminum, etc.

In preferred embodiments, the sodium salt is selected from at least one of: sodium perchlorate ($NaClO_4$), sodium hexafluorophosphate ($NaPF_6$), sodium tetrafluoroborate ($NaBF_4$), sodium trifluoromethanesulfonate (NaFSI) or sodium bis-trifluoromethanesulfonate (NaTFSI).

The above-mentioned analysis is only based on the impact of each parameter or multiple parameters present individually on the battery. However, in an actual battery application process, parameters such as the mass percentage content a of the compound represented by the formula 1 in the non-aqueous electrolyte, the mass percentage content m of the carboxylate in the non-aqueous electrolyte and the mass percentage content n of the electrolyte salt in the non-aqueous electrolyte are associated within a certain extent. Through a large number of tests, the inventor summarizes that a relational expression of $0.02 \leq an/m \leq 9$, which may be effectively serve as a basis for screening a secondary battery with a high energy density, a fast charge performance and an excellent cycle performance.

In some embodiments, the compound represented by the formula 1 is selected from one or more of following compounds 1-22:

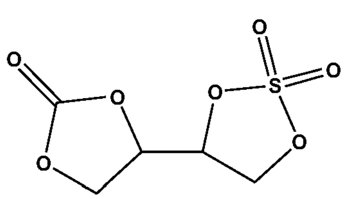
Compound 1

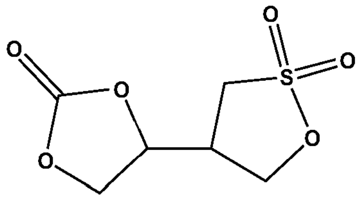
Compound 2

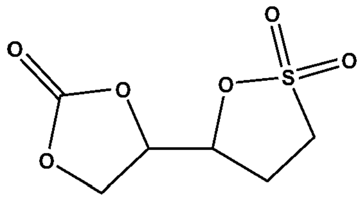
Compound 3

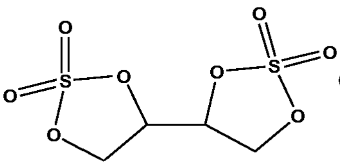
Compound 4

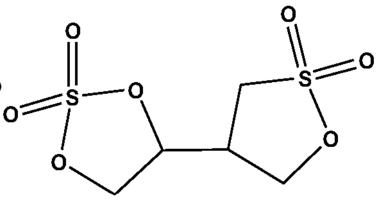
Compound 5

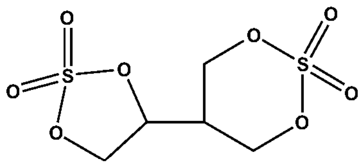
Compound 6

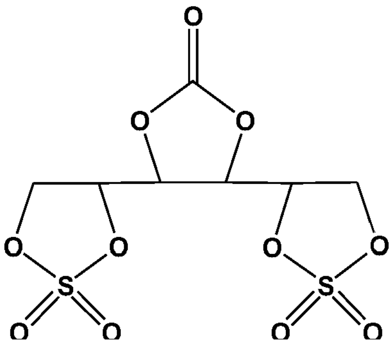
Compound 7

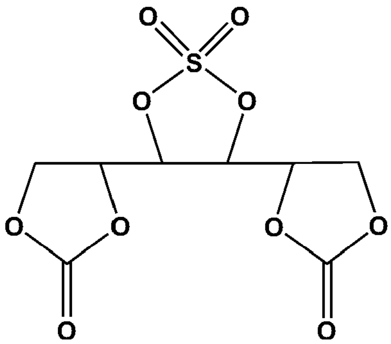
Compound 8

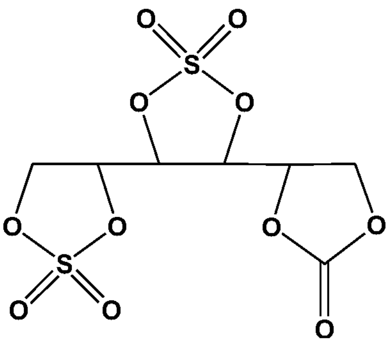
Compound 9

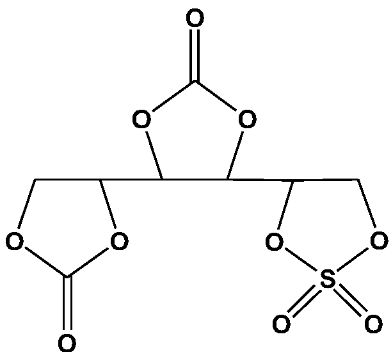
Compound 10

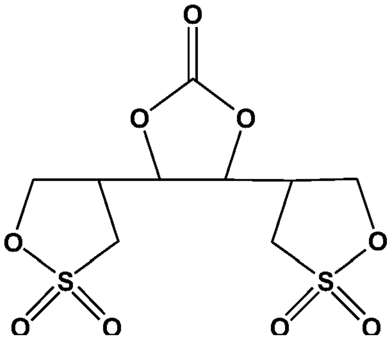

Compound 11

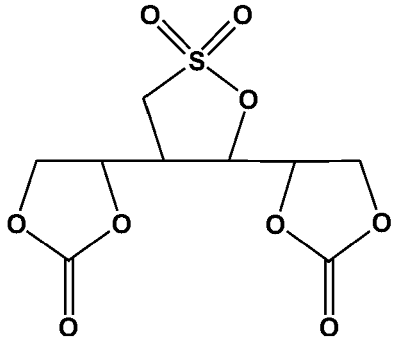

Compound 12

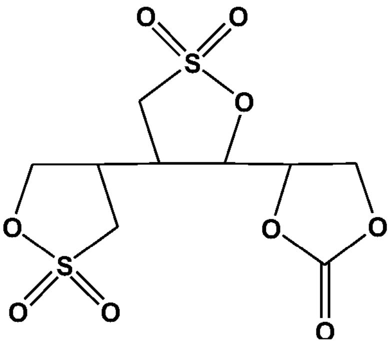

Compound 13

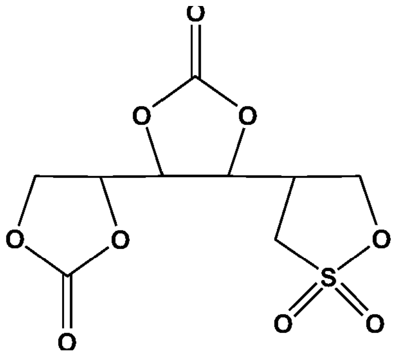

Compound 14

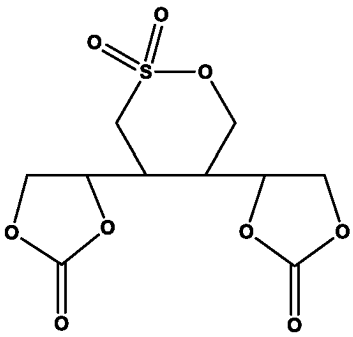

Compound 15

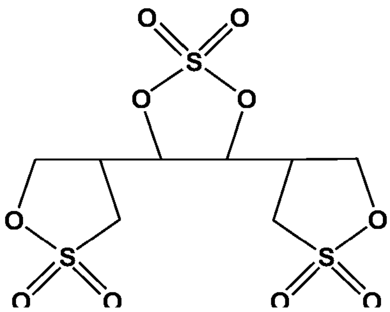

Compound 16

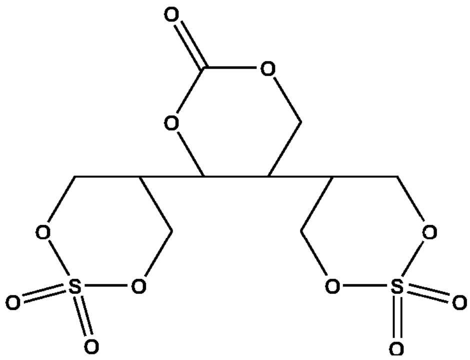

Compound 17

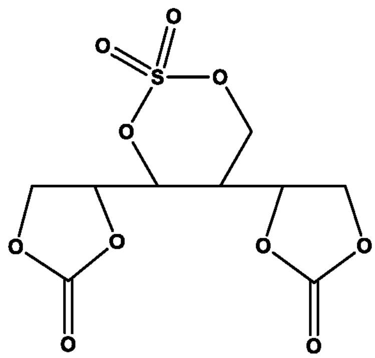

Compound 18

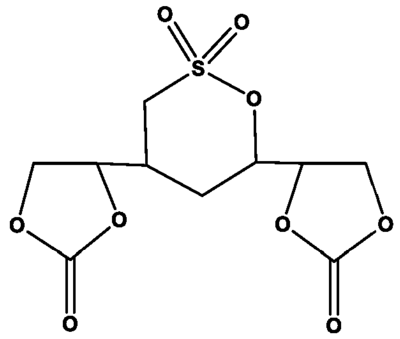

Compound 19

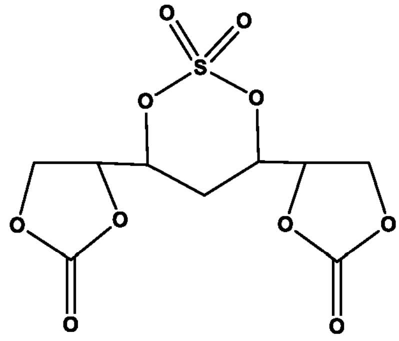

Compound 20

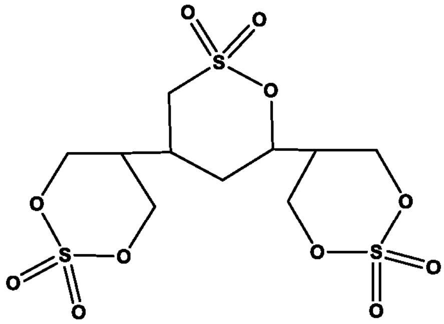

Compound 21

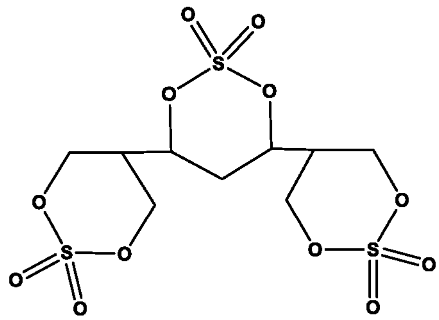

Compound 22

It is to be noted that the above-mentioned compounds are only preferred compounds of the present invention, and do not represent limitations on the present invention.

Those skilled in the art may know the preparation method of the above-mentioned compounds according to the general knowledge in the field of chemical synthesis when they know a structural formula of the compound represented by the formula 1. For example, compound 7 may be prepared by a method as follows:

Organic solvents such as sorbitol, dimethyl carbonate, methanol alkaline substance catalyst potassium hydroxide, DMF, and the like are placed in a reaction vessel. After reaction for several hours under heating conditions, a certain amount of oxalic acid is added to adjust the pH to be neutral. Filtration and recrystallization are performed to obtain an intermediate product 1. The intermediate product 1, carbonate, thionyl chloride, and the like are subjected to an esterification reaction at high temperature to obtain an intermediate product 2. An oxidizing agent such as sodium periodate is used to oxidize the intermediate product 2 to obtain the compound 7.

In some embodiments, the carboxylate includes one or more of: methyl acetate, ethyl acetate, propyl acetate, methyl propionate, ethyl propionate, propyl propionate, 2,2-difluoroethyl acetate, methyl butyrate, methyl isobutyrate, methyl trimethylacetate and ethyl trimethylacetate.

In some embodiments, the non-aqueous organic solvent further includes one or more of: ether solvents, nitrile solvents and carbonate solvents.

In some embodiments, the ether solvents include cyclic ethers or linear ethers, preferably linear ethers having 3 to 10 carbon atoms and cyclic ethers having 3 to 6 carbon atoms. The cyclic ethers may specifically be, but are not limited to one or more of: 1,3-dioxolane (DOL), 1,4-dioxane (DX), crown ethers, tetrahydrofuran (THF), 2-methyltetrahydrofuran (2-$CH_3$-THF), and 2-trifluoromethyltetrahydrofuran (2-$CF_3$-THF). The linear ethers may specifically be, but are not limited to dimethoxymethane, diethoxymethane, ethoxymethoxymethane, ethylene glycol di-n-propyl ether, ethylene glycol di-n-butyl ether and diethyleneglycoldimethylether. Since solvability of the linear ethers and lithium ions is high and ion dissociation may be improved, dimethoxymethane, diethoxymethane and ethoxymethoxymethane with a small viscosity and a high ionic conductivity are particularly preferred. Ether compounds may be used alone, or in combination of two or more in any combination and ratio. An addition amount of the ether compounds is not particularly limited, and it is arbitrary within a range that does not significantly destroy the effect of the high compaction lithium-ion battery of the present invention. When a volume ratio of the non-aqueous organic solvent is 100%, a volume ratio of the ether compounds is usually 1% or more, preferably 2% or more, more preferably 3% or more, and in addition, the volume ratio of the ether compounds is generally 30% or less, preferably 25% or less, and more preferably 20% or less. When two or more ether

compounds are used in combination, a total amount of the ether compounds is within the above-mentioned range. When an addition amount of the ether compounds is within the above-mentioned preferred range, it is easy to ensure an effect of improving the ionic conductivity by increasing a lithium ion dissociation degree and reducing the viscosity of the linear ethers. In addition, when a negative electrode active material is a carbon material, a phenomenon of co-intercalating of the linear ethers and the lithium ions may be inhibited, so that input and output characteristics and charge and discharge rate characteristics may reach appropriate ranges.

In some embodiments, the nitrile solvents may specifically be, but are not limited to one or more of: acetonitrile, glutaronitrile, and malononitrile.

In some embodiments, the carbonate solvents include cyclic carbonates or linear carbonates. The cyclic carbonates may specifically be, but are not limited to one or more of: ethylene carbonate (EC), propylene carbonate (PC), γ-butyrolactone (GBL), and butylene carbonate (BC). The linear carbonates may specifically be, but are not limited to one or more of: dimethyl carbonate (DMC), ethyl methyl carbonate (EMC), diethyl carbonate (DEC), and dipropyl carbonate (DPC). A content of the cyclic carbonates is not particularly limited, and it is arbitrary within a range that does not significantly destroy the effect of the secondary battery of the present invention. However, when the cyclic carbonate is used alone, a volume ratio of a lower limit of a content of the cyclic carbonate is usually 3% or more, preferably 5% or more, relative to a total amount of the non-aqueous organic solvent in the non-aqueous electrolyte. By setting this range, it is possible to avoid a decrease in conductivity due to a decrease in the dielectric constant of the non-aqueous electrolyte, and it is easy to make high current discharge characteristics, stability relative to the negative electrode and cycle characteristics of a non-aqueous electrolyte battery reach a good range. A volume ratio of an upper limit of a content of the cyclic carbonate is usually 90% or less, preferably 85% or less, and more preferably 80% or less. By setting this range, oxidation/reduction resistance of the non-aqueous electrolyte may be improved, thus contributing to improve the stability during high temperature storage. A content of the linear carbonates is not particularly limited, and a volume ratio of the linear carbonates is usually 15% or more, preferably 20% or more, and more preferably 25% or more, relative to a total amount of the non-aqueous organic solvent in the non-aqueous electrolyte. In addition, a volume ratio of the linear carbonates is usually 90% or less, preferably 85% or less, and more preferably 80% or less. By making the content of the linear carbonates in the above-mentioned range, it is easy to make the viscosity of the non-aqueous electrolyte reach an appropriate range and inhibit the decrease of the ionic conductivity, thus contributing to make output characteristics of the non-aqueous electrolyte battery reach a good range. When two or more linear carbonates are used in combination, a total amount of the linear carbonates is within the above-mentioned range.

In some embodiments, it is also preferable to use linear carbonates having fluorine atom(s) (hereinafter referred to as "fluorinated linear carbonates"). The number of the fluorine atoms in the fluorinated linear carbonate is not particularly limited as long as it is 1 or more, but it is usually 6 or less, preferably 4 or less. When the fluorinated linear carbonate has a plurality of fluorine atoms, these fluorine atoms may be bonded to a same carbon, or may be bonded to different carbons. Examples of the fluorinated linear carbonates include fluorinated dimethyl carbonate derivatives, fluorinated methyl ethyl carbonate derivatives, and fluorinated diethyl carbonate derivatives.

In some embodiments, sulfone solvents include cyclic sulfones and linear sulfones. Preferably, when the sulfone solvents is the cyclic sulfones, the cyclic sulfones is usually a compound having 3 to 6 carbon atoms, preferably 3 to 5 carbon atoms. When the sulfone solvents is the linear sulfones, the linear sulfones is usually a compound having 2 to 6 carbon atoms, preferably 2 to 5 carbon atoms. An addition amount of the sulfone solvents is not particularly limited, and it is arbitrary within a range that does not significantly destroy the effect of the secondary battery of the present invention. A volume ratio of the sulfone solvents is usually 0.3% or more, preferably 0.5% or more, more preferably 1% or more, and in addition, the volume ratio of the sulfone solvents is generally 40% or less, preferably 35% or less, and more preferably 30% or less, relative to a total amount of non-aqueous organic solvent in non-aqueous electrolyte. When two or more sulfone solvents are used in combination, a total amount of the sulfone solvents is within the above-mentioned range. When the addition amount of the sulfone solvents is within the above-mentioned range, it tends to obtain an electrolyte with excellent high temperature storage stability.

In some embodiments, the non-aqueous electrolyte further includes an auxiliary additive. The auxiliary additive includes at least one of: a cyclic sulfate compound, a sultone compound, a cyclic carbonate compound, an unsaturated phosphate compound, a nitrile compound and a lithium oxalate borate.

In preferred embodiments, the cyclic sulfate compound is selected from at least one of: ethylene sulfate, propene sulfate or methyl ethylene sulfate.

The sultone compound is selected from at least one of: 1,3-propane sultone, 1,4-butane sultone or 1,3-propene sultone.

The cyclic carbonate compound is selected from at least one of: vinylene carbonate, vinyl ethylene carbonate, fluoroethylene carbonate or a compound represented by formula 2,

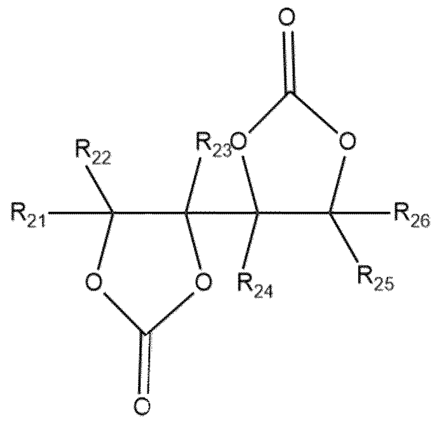

Formula 2

in the formula 2, $R_{21}$, $R_{22}$, $R_{23}$, $R_{24}$, $R_{25}$ and $R_{26}$ are each independently selected from one of: a hydrogen atom, a halogen atom or a C1-C5 group.

The unsaturated phosphate compound is selected from at least one of compounds represented by formula 3:

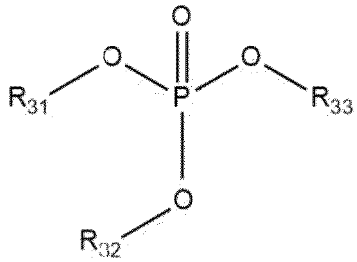

Formula 3

in the formula 3, $R_{31}$, $R_{32}$ and $R_{33}$ are each independently selected from a C1-C5 saturated hydrocarbon group, an unsaturated hydrocarbon group, a halogenated hydrocarbon group and $-Si(C_mH_{2m+1})_3$, where m is a natural number of 1 to 3, and at least one of $R_{31}$, $R_{32}$ and $R_{33}$ is an unsaturated hydrocarbon group.

In preferred embodiments, the unsaturated phosphate compound may be at least one of: tripropargyl phosphate, dipropargyl methyl phosphate, dipropargyl ethyl phosphate, dipropargyl propyl phosphate, dipropargyl trifluoromethyl phosphate, dipropargyl-2,2,2-trifluoroethyl phosphate, dipropargyl-3,3,3-trifluoropropyl phosphate, dipropargyl hexafluoroisopropyl phosphate, triallyl phosphate, diallyl methyl phosphate, diallyl ethyl phosphate, diallyl propyl phosphate, diallyl trifluoromethyl phosphate, diallyl-2,2,2-trifluoroethyl phosphate, diallyl-3,3,3-trifluoropropyl phosphate, diallyl hexafluoroisopropyl phosphate.

The nitrile compound is selected from one or more of: succinonitrile, glutaronitrile, ethylene glycol bis(propionitrile) ether, hexanitrile, adiponitrile, pimelonitrile, suberonitrile, azelanitrile and sebaconitrile.

The lithium oxalate borate is selected from one or more of: $Li[B(C_2O_4)_2]$ and $Li[B(C_2O_4)F_2]$.

In other embodiments, the auxiliary additive may further include other additives that may improve a performance of the battery, for example, additives that may improve a safety performance of the battery, specifically for example, flame retardant additives such as fluorophosphate and cyclophosphazene, or anti-overcharge additives such as tert-amylbenzene and tert-butylbenzene.

In some embodiments, the auxiliary additive is added in an amount of 0.01% to 30% based on a total mass of the non-aqueous electrolyte as 100%.

It is to be noted that, unless otherwise specified, in general, any optional substance in the auxiliary additive in the non-aqueous electrolyte is added in an amount of 10% or less, preferably 0.1 to 5%, and more preferably 0.1% to 2%. Specifically, any optional substance in the auxiliary additive may be added in an amount of 0.05%, 0.08%, 0.1%, 0.5%, 0.8%, 1%, 1.2%, 1.5%, 1.8%, 2%, 2.2%, 2.5%, 2.8%, 3%, 3.2%, 3.5%, 3.8%, 4%, 4.5%, 5%, 5.5%, 6%, 6.5%, 7%, 7.5%, 7.8%, 8%, 8.5%, 9%, 9.5%, and 10%.

In some embodiments, when the auxiliary additive is selected from fluoroethylene carbonate, the fluoroethylene carbonate is added in an amount of 0.05% to 30% based on a total mass of the non-aqueous electrolyte as 100%.

Another embodiment of the present invention provides a secondary battery. The secondary battery includes a positive electrode, a negative electrode, and a non-aqueous electrolyte as described above. The positive electrode includes a positive electrode material layer, and a compaction density of the positive electrode material layer is 3.5g/cm$^3$ or more. The negative electrode includes a negative electrode material layer, and a compaction density of the negative electrode material layer is 1.5g/cm$^3$ or more.

In preferred embodiments, the compaction density of the positive electrode material layer is 3.8 to 4.15g/cm$^3$.

In preferred embodiments, the compaction density of the negative electrode material layer is 1.65 to 1.85g/cm$^3$.

In some embodiments, a porosity of the positive electrode material and the negative electrode material is 50% or less.

In preferred embodiments, a porosity of the positive electrode material and the negative electrode material is 10% to 35%.

In some embodiments, a highest cutoff charge voltage of the secondary battery is ≥ 4.4V

In some embodiments, the positive electrode material layer includes a positive electrode active material. A type and a content of the positive electrode active material are not particularly limited, and may be selected according to actual needs, as long as it is a positive electrode active material or a conversion positive electrode material capable of reversibly intercalating/deintercalating metal ions, such as lithium ion, sodium ion, potassium ion, magnesium ion, zinc ion, aluminum ion, etc..

In preferred embodiments, the battery is a lithium-ion battery. A positive electrode active material of the lithium-ion battery may be selected from one or more of: $LiFe_{1-x'}M'_{x'}PO_4$, $LiMn_{2-y'}M_{y'}O_4$ and $LiNi_xCo_yMn_zM_{1-x-y-z}O_2$, where $0 \le x' < 1$, $0 \le y' \le 1$, $0 \le y \le 1$, $0 \le x \le 1$, $0 \le z \le 1$, $x+y+z \le 1$. M' is selected from one or more of: Mn, Mg, Co, Ni, Cu, Zn, Al, Sn, B, Ga, Cr, Sr, V or Ti. M is selected from one or more of: Fe, Co, Ni, Mn, Mg, Cu, Zn, Al, Sn, B, Ga, Cr, Sr, V or Ti. The positive electrode active material may also be selected from one or more of: sulfide, selenide and halide. More preferably, the positive electrode active material may be selected from one or more of: $LiCoO_2$, $LiFePO_4$, $LiFe_{0.8}Mn_{0.2}PO_4$, $LiMn_2O_4$, $LiNi_{0.5}Co_{0.2}Mn_{0.3}O_2$, $LiNi_{0.6}Co_{0.2}Mn_{0.2}O_2$, $LiNi_{0.8}Co_{0.1}Mn_{0.1}O_2$, $LiNi_{0.5}Co_{0.2}Mn_{0.2}Al_{0.1}O_2$ and $LiNi_{0.5}Co_{0.2}Al_{0.3}O_2$.

In preferred embodiments, the secondary battery is a sodium ion battery. A positive electrode active material of the sodium ion battery may be selected from one or more of: a metal sodium, a carbon material, an alloy material, a transition metal oxide, a transition metal sulfide, a phosphorus-based material, a titanate material and a Prussian blue material. The carbon material may be selected from one or more of: graphite, soft carbon and hard carbon. The alloy material may be selected from alloy materials composed of at least two of Si, Ge, Sn, Pb and Sb. The alloy material may also be selected from alloy materials composed of at least one of: Si, Ge, Sn, Pb and Sb, and C. A chemical formula of the transition metal oxide and the transition metal sulfide is $M1_xN_y$. M1 may be selected from one or more of: Fe, Co, Ni, Cu, Mn, Sn, Mo, Sb and V. N may be selected from O or S. The phosphorus-based material may be selected from one or more of: red phosphorus, white phosphorus and black phosphorus. The titanate material may be selected from one or more of: $Na_2Ti_3O_7$, $Na_2Ti_6O_{13}$, $Na_4Ti_5O_{12}$, $Li_4Ti_5O_{12}$ and $NaTi_2(PO_4)_3$. A molecular formula of the Prussian blue material is $Na_xM[M'(CN)_6]_y \cdot zH_2O$, where M is a transition metal, and M' is a transition metal, $0<x\le 2$, $0.8\le y<1$, $0<z\le 20$.

In some embodiments, the positive electrode further includes a positive electrode current collector. The positive electrode material layer covers a surface of the positive electrode current collector.

The positive electrode current collector is selected from metal materials that may conduct electrons. Preferably, the positive electrode current collector includes one or more of: Al, Ni, tin, copper and stainless steel. In a more preferred embodiment, the positive electrode current collector is selected from aluminum foil.

In some embodiments, the positive electrode active material layer further includes a positive electrode binder and a positive electrode conductive agent. The positive electrode active material, the positive electrode binder and the positive electrode conductive agent are blended to obtain the positive electrode material layer.

The positive electrode binder includes one or more of: thermoplastic resins such as polyvinylidene fluoride, vinylidene fluoride copolymers, polytetrafluoroethylene, vinylidene fluoride-hexafluoropropylene copolymers, tetrafluoroethylene-hexafluoropropylene copolymers, tetrafluoroethylene-perfluoroalkyl vinyl ether copolymers, ethylene-tetrafluoroethylene copolymers, vinylidene fluoride-tetrafluoroethylene copolymers, vinylidene fluoride-trifluoroethylene copolymers, vinylidene fluoride-trichloroethylene copolymers, vinylidene fluoride-fluoroethylene copolymers, vinylidene fluoride-hexafluoropropylene-tetrafluoroethylene copolymers, thermoplastic polyimides, polyethylene, polypropylene and so on; acrylic resins; and styrene butadiene rubbers.

The positive electrode conductive agent includes one or more of: conductive carbon black, conductive carbon spheres, conductive graphite, conductive carbon fibers, carbon nanotubes, graphene or reduced graphene oxide.

In some embodiments, the negative electrode includes the negative electrode material layer. The negative electrode material layer includes a negative electrode active material. The negative electrode active material includes one or more of: a carbon-based negative electrode, a tin-based negative electrode, a silicon-based negative electrode, a lithium negative electrode, a sodium negative electrode, a potassium negative electrode, a magnesium negative electrode, a zinc negative electrode and an aluminum negative electrode. The carbon-based negative electrode may include graphite, hard carbon, soft carbon, graphene, mesophase carbon microspheres, etc. The silicon-based negative

electrode may include one or more of: a silicon material, a silicon oxide, a silicon-carbon composite material and a silicon alloy material. The tin-based negative electrode may include tin, tin carbon, tin oxide and tin metal compounds. The lithium negative electrode may include metallic lithium or lithium alloy. The lithium alloy may be at least one of: lithium silicon alloy, lithium sodium alloy, lithium potassium alloy, lithium aluminum alloy, lithium tin alloy and lithium indium alloy.

In some embodiments, the negative electrode further includes a negative electrode current collector. The negative electrode material layer covers a surface of the negative electrode current collector. A material of the negative electrode current collector may be the same as that of the positive electrode current collector, which will not be elaborated herein.

In some embodiments, the negative electrode material layer further includes a negative electrode binder and a negative electrode conductive agent. The negative electrode active material, the negative electrode binder and the negative electrode conductive agent are blended to obtain the negative electrode material layer. The negative electrode binder and the negative electrode conductive agent may be the same as the positive electrode binder and the positive electrode conductive agent, respectively, which will not be elaborated herein.

In some embodiments, the battery further includes a separator, and the separator is located between the positive electrode and the negative electrode.

The separator may be an existing conventional separator, and may be a ceramic separator, a polymer separator, a nonwoven fabric, an inorganic-organic composite separator, etc., including but not limited to separators, such as single-layer polypropylene (PP), single-layer polyethylene (PE), double-layer PP/PE, double-layer PP/PP, triple-layer PP/PE/PP, and the like.

The present invention is further illustrated by way of examples below.

The compounds involved in the following examples and comparative examples are shown in the table below:

| | | | |
|---|---|---|---|
| Compound 1 | 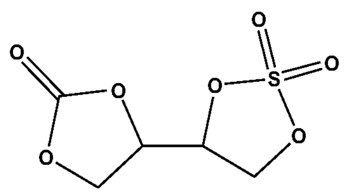 | Compound 3 | 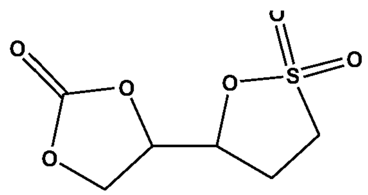 |
| Compound 4 | 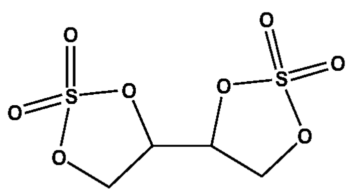 | Compound 7 | 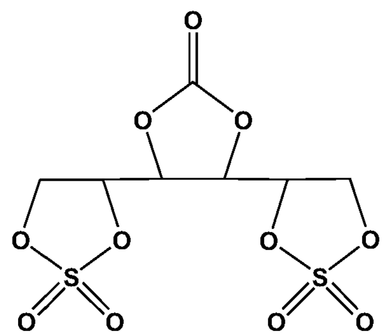 |
| Compound 8 | 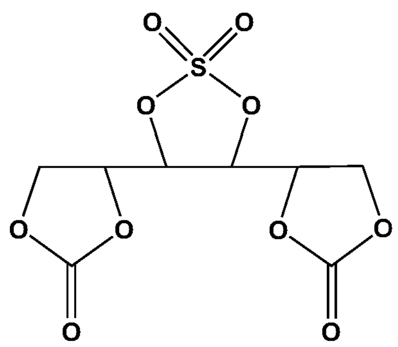 | Compound 20 | 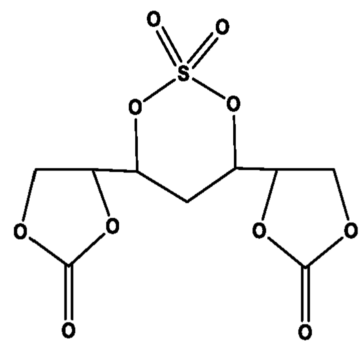 |

Table 1. Design of Parameters of Examples and Comparative Examples

| | Type of compound represented by formula 1 | Content a of compound represented by formula 1/% | Other additives and their contents | Type of carboxylate | content m of carboxylate /% | content n of lithium salt/% | an/m |
|---|---|---|---|---|---|---|---|
| Example 1 | Compound 1 | 0.01 | - | propyl propionate | 5.0 | 10.0 | 0.02 |
| Example 2 | Compound 1 | 0.05 | - | propyl propionate | 8.0 | 12.0 | 0.08 |
| Example 3 | Compound 1 | 0.1 | - | propyl propionate | 20.0 | 8.0 | 0.04 |
| Example 4 | Compound 1 | 0.2 | | propyl propionate | 25.0 | 12.5 | 0.10 |
| Example 5 | Compound 1 | 0.5 | - | propyl propionate | 20.0 | 12.0 | 0.30 |
| Example 6 | Compound 1 | 0.5 | - | propyl propionate | 50.0 | 12.0 | 0.12 |
| Example 7 | Compound 1 | 0.8 | - | propyl propionate | 40.0 | 13.5 | 0.27 |
| Example 8 | Compound 1 | 1.0 | - | propyl propionate | 10.0 | 25.0 | 2.50 |
| Example 9 | Compound 2 | 1.0 | | propyl propionate | 20.0 | 15.0 | 0.75 |
| Example 10 | Compound 1 | 1.0 | - | propyl propionate | 30.0 | 15.0 | 0.50 |
| Example 11 | Compound 1 | 1.0 | - | propyl propionate | 35.0 | 25.0 | 0.71 |
| Example 12 | Compound 1 | 1.0 | - | propyl propionate | 50.0 | 20.0 | 0.40 |
| Example 13 | Compound 1 | 1.2 | - | propyl propionate | 24.0 | 12.0 | 0.60 |
| Example 14 | Compound 1 | 1.5 | - | propyl propionate | 30.0 | 10.0 | 0.50 |
| Example 15 | Compound 1 | 1.8 | - | propyl propionate | 30.0 | 20.0 | 1.20 |
| Example 16 | Compound 1 | 2.0 | - | propyl propionate | 10.0 | 20.0 | 4.00 |
| Example 17 | Compound 1 | 2.5 | - | propyl propionate | 15.0 | 20.0 | 3.33 |
| Example 18 | Compound 1 | 2.5 | - | propyl propionate | 50.0 | 25.0 | 1.25 |
| Example 19 | Compound 1 | 2.5 | - | propyl propionate | 5.0 | 15.0 | 7.50 |
| Example 20 | Compound 1 | 2.5 | - | propyl propionate | 10.0 | 20.0 | 5.00 |
| Example 21 | Compound 1 | 3.0 | - | propyl propionate | 25.0 | 18.0 | 2.160 |

(continued)

| | Type of compound represented by formula 1 | Content a of compound represented by formula 1/% | Other additives and their contents | Type of carboxylate | content m of carboxylate /% | content n of lithium salt/% | an/m |
|---|---|---|---|---|---|---|---|
| Example 22 | Compound 1 | 3.0 | - | propyl propionate | 20.0 | 20.0 | 3.00 |
| Example 23 | Compound 1 | 4.0 | - | propyl propionate | 30.0 | 15.0 | 2.00 |
| Example 24 | Compound 1 | 4.5 | - | propyl propionate | 70.0 | 20.0 | 1.29 |
| Example 25 | Compound 1 | 5.0 | - | propyl propionate | 15.0 | 20.0 | 6.67 |
| Example 26 | Compound 1 | 5.0 | - | propyl propionate | 50.0 | 25.0 | 2.50 |
| Example 27 | Compound 1 | 3.0 | - | propyl propionate | 5.0 | 15.0 | 9.00 |
| Example 28 | Compound 1 | 1.5 | - | propyl propionate | 5.0 | 15.0 | 4.50 |
| Example 29 | Compound 1 | 1.5 | - | propyl propionate | 10.0 | 25.0 | 3.75 |
| Example 30 | Compound 1 | 5.0 | - | propyl propionate | 60.0 | 25.0 | 2.08 |
| Example 31 | Compound 3 | 1.0 | - | propyl propionate | 30 | 15 | 0.50 |
| Example 32 | Compound 4 | 1.0 | - | propyl propionate | 30 | 15 | 0.50 |
| Example 33 | Compound 7 | 1.0 | - | propyl propionate | 30 | 15 | 0.50 |
| Example 34 | Compound 8 | 1.0 | - | propyl propionate | 30 | 15 | 0.50 |
| Example 35 | Compound 20 | 1.0 | - | propyl propionate | 30 | 15 | 0.50 |
| Example 36 | Compound 1 | 1.0 | - | ethyl propionate | 30 | 15 | 0.50 |
| Example 37 | Compound 1 | 1.0 | - | ethyl acetate | 30 | 15 | 0.50 |
| Example 38 | Compound 1 | 1.0 | - | 2,2-difluoroethyl acetate | 30 | 15 | 0.50 |
| Example 39 | Compound 1 | 1.0 | PS 0.5% | propyl propionate | 30 | 15 | 0.50 |
| Example 40 | Compound 1 | 1.0 | DTD 1% | propyl propionate | 30 | 15 | 0.50 |
| Example 41 | Compound 1 | 1.0 | tripropargyl phosphate 0.5% | propyl propionate | 30 | 15 | 0.50 |
| Example 42 | Compound 1 | 1.0 | succinonitrile 1% | propyl propionate | 30 | 15 | 0.50 |

(continued)

| | Type of compound represented by formula 1 | Content a of compound represented by formula 1/% | Other additives and their contents | Type of carboxylate | content m of carboxylate /% | content n of lithium salt/% | an/m |
|---|---|---|---|---|---|---|---|
| Example 43 | Compound 1 | 1.0 | 0.5% LiODFB | propyl propionate | 30 | 15 | 0.50 |
| Comparative Example 1 | Compound 1 | 0.1 | - | propyl propionate | 60 | 10 | 0.02 |
| Comparative Example 2 | Compound 1 | 0.05 | - | propyl propionate | 60 | 15 | 0.01 |
| Comparative Example 3 | Compound 1 | 5.0 | - | propyl propionate | 10 | 20 | 10.00 |
| Comparative Example 4 | Compound 1 | 2.0 | - | propyl propionate | 5 | 25 | 10.00 |
| Comparative Example 5 | Compound 1 | 3.0 | - | propyl propionate | 5 | 20 | 12.00 |
| Comparative Example 6 | Compound 1 | 1.5 | - | propyl propionate | 10 | 5 | 0.75 |
| Comparative Example 7 | - | - | - | propyl propionate | 30 | 15 | - |
| Comparative Example 8 | Compound 1 | 3.0 | - | propyl propionate | 3 | 15 | 15.00 |
| Comparative Example 9 | Compound 1 | 3.0 | - | propyl propionate | 80 | 15 | 0.56 |
| Comparative Example 10 | Compound 1 | 0.009 | - | propyl propionate | 8 | 20 | 0.02 |
| Comparative Example 11 | Compound 1 | 6.0 | - | propyl propionate | 30 | 15 | 3.00 |
| Comparative Example 12 | Compound 1 | 5.0 | - | propyl propionate | 10 | 5 | 2.50 |
| Comparative Example 13 | Compound 1 | 1.0 | - | propyl propionate | 30 | 28 | 0.93 |

Example 1

Example 1 is used to illustrate a battery and a preparation method thereof disclosed in the present invention, including steps as follows:

1) Preparation of electrolyte: solvent ethylene carbonate (EC), diethyl carbonate (DEC) and carboxylate were mixed, and then lithium hexafluorophosphate ($LiPF_6$) and an additive were added. Contents of the carboxylate, the lithium hexafluorophosphate and the additive in the electrolyte are shown in Table 1, and the contents are calculated as a percentage of the total mass of the electrolyte.
2) Preparation of positive electrode sheet

A positive electrode active material $LiCoO_2$, conductive carbon black Super-P and binder polyvinylidene fluoride (PVDF) were mixed in a mass ratio of 93:4:3, and then dispersed in N-methyl-2-pyrrolidone (NMP) to obtain a positive electrode slurry. The slurry was evenly coated on both sides of aluminum foil, dried, calendered and vacuum dried, and an aluminum lead-out wire was welded with an ultrasonic welder to obtain a positive electrode sheet. A thickness of the positive electrode sheet is 80 to 150μm, and a compaction density of the positive electrode sheet is $4.13g/cm^3$.

3) Preparation of negative electrode sheet

A negative electrode active material artificial graphite, conductive carbon black Super-P, binder styrene-butadiene rubber (SBR) and carboxymethyl cellulose (CMC) were mixed in a mass ratio of 94:1 :2.5:2.5, and then dispersed in deionized water to obtain a negative electrode slurry. The slurry was coated on both sides of copper foil, dried, and rolled, and a nickel lead-out wire was welded with an ultrasonic welder to obtain a negative electrode sheet with a compaction density of 1.81g/cm$^3$.

4) Preparation of battery cell

A polyethylene microporous membrane with a thickness of 20μm was placed between the positive electrode sheet and the negative electrode sheet as a separator, and then a sandwich structure composed of the positive electrode sheet, the negative electrode sheet and the separator was wound. Then, a wound body was flattened and placed in an aluminum-plastic film. After lead-out wires of the positive and negative electrodes were led out, respectively, the aluminum-plastic film was hot-pressed and sealed to obtain the battery cell to be injected with liquid.

5) Liquid injection and formation of battery cell

In a glove box with a dew point controlled below -40°C, an electrolyte as prepared above was injected into the battery cell via a liquid injection hole, and an amount of the electrolyte was ensured to fill a gap in the battery cell. Then, formation was performed according to the following steps: charging at a constant current of 0.05C for 180min, charging at a constant current of 0.1C for 180min, shaping and sealing after standing for 24h, then further charging at a constant current of 0.2C to a cutoff voltage of 4.50V, standing at room temperature for 24h, and discharging at a constant current of 0.2C to 3.0V.

Examples 2 to 43

Examples 2 to 43 are used to illustrate a battery and a preparation method thereof disclosed in the present invention, including most of the steps in Example 1, excepting that an electrolyte is prepared by adding components shown in Table 1.

Comparative Examples 1 to 13

Comparative Examples 1 to 13 are used to compare and illustrate a battery and a preparation method thereof disclosed in the present invention, including most of the steps in Example 1, excepting that an electrolyte is prepared by adding components shown in Table 1.

Performance testing

The lithium-ion batteries prepared above were subjected to the following performance tests.

Under a condition of 25°C, a conductivity of the non-aqueous electrolyte was tested. It was found that conductivities of Examples 1 to 43, Comparative Examples 1 to 5, Comparative Examples 7 to 11 and Comparative Example 13 were all 5mS/cm or more, and those of Comparative Examples 6 and 12 were 5mS/cm or less.

Under a condition of 45°C, the lithium-ion battery was charged at a constant current of 3C to 4.50V, charged at a constant voltage of 4.50V until the current drops to 0.05C, and then discharged at a constant current of 3C to 3.0V. This was cycled for 400 cycles. A discharge capacity, discharge DCIR, of a first cycle and a discharge capacity, discharge DCIR, of a 400$^{th}$ cycle were recorded. A capacity retention rate and a DCIR growth rate were calculated according to the following formula:

Capacity retention rate (%) = (the discharge capacity of the 400$^{th}$ cycle ÷ the discharge capacity of the first cycle) × 100%;

DCIR growth rate (%) = (the discharge DCIR of the 400$^{th}$ cycle - the discharge DCIR of the first cycle) / the discharge DCIR of the first cycle.

(1) Test results obtained in Examples 1 to 30 and Comparative Examples 1 to 13 are shown in Table 2.

Table 2

| Group | Capacity retention rate of 400 cycles at 45°C at 3C/3C (%) | Internal resistance growth rate of 400 cycles at 45°C at 3C/3C (%) |
|---|---|---|
| Example 1 | 75.3 | 72.9 |
| Example 2 | 75.7 | 70.1 |
| Example 3 | 76.0 | 72.8 |
| Example 4 | 82.2 | 55.2 |
| Example 5 | 83.2 | 52.1 |
| Example 6 | 85.3 | 50.1 |
| Example 7 | 86.1 | 46.8 |
| Example 8 | 86.2 | 45.3 |
| Example 9 | 88.9 | 41.2 |
| Example 10 | 89.1 | 40.1 |
| Example 11 | 88.4 | 42.9 |
| Example 12 | 87.6 | 46.5 |
| Example 13 | 88.3 | 43.0 |
| Example 14 | 86.2 | 45.6 |
| Example 15 | 86.7 | 45.1 |
| Example 16 | 77.6 | 49.9 |
| Example 17 | 80.7 | 53.1 |
| Example 18 | 85.3 | 49.8 |
| Example 19 | 76.3 | 70.9 |
| Example 20 | 76.9 | 69.8 |
| Example 21 | 83.6 | 81.6 |
| Example 22 | 83.2 | 52.3 |
| Example 23 | 85.1 | 58.2 |
| Example 24 | 82.6 | 57.5 |
| Example 25 | 74.9 | 75.2 |
| Example 26 | 83.4 | 53.6 |
| Example 27 | 75.4 | 73.8 |
| Example 28 | 76.2 | 71.4 |
| Example 29 | 78.2 | 51.0 |
| Example 30 | 83.2 | 52.6 |
| Comparative Example 1 | 60.1 | 120.5 |
| Comparative Example 2 | 60.3 | 130.2 |
| Comparative Example 3 | 63.2 | 110.2 |
| Comparative Example 4 | 64.3 | 108.9 |
| Comparative Example 5 | 65.2 | 103.5 |
| Comparative Example 6 | 44.2 | 204.5 |
| Comparative Example 7 | 56.2 | 150.3 |
| Comparative Example 8 | 68.9 | 89.4 |
| Comparative Example 9 | 64.9 | 99.7 |

(continued)

| Group | Capacity retention rate of 400 cycles at 45°C at 3C/3C (%) | Internal resistance growth rate of 400 cycles at 45°C at 3C/3C (%) |
|---|---|---|
| Comparative Example 10 | 63.5 | 105.2 |
| Comparative Example 11 | 60.1 | 120.6 |
| Comparative Example 12 | 45.2 | 190.8 |
| Comparative Example 13 | 43.6 | 200.9 |

From the test results of Examples 1 to 30 and Comparative Examples 1 to 13, it may be seen that the mass percentage content a of the compound represented by formula 1 in the non-aqueous electrolyte, the mass percentage content m of the carboxylate in the non-aqueous electrolyte and the mass percentage content n of the electrolyte salt in the non-aqueous electrolyte has an obvious association. When the mass percentage content a of the compound represented by the formula 1 in the non-aqueous electrolyte, the mass percentage content m of the carboxylate in the non-aqueous electrolyte and the mass percentage content n of the electrolyte salt in the non-aqueous electrolyte satisfy relational expressions $0.02 \leq an/m \leq 9$, $0.01\% \leq a \leq 5\%$, $5\% \leq m \leq 70\%$, and $8\% \leq n \leq 25\%$, an obtained lithium-ion battery has a higher cycle capacity retention rate and a lower impedance growth rate under a condition of large current fast charging. It is speculated that due to the fact that the compound represented by the formula 1 participates in film formation on the positive and the negative electrodes simultaneously, a formed passivation film may effectively inhibit a decomposition reaction of the carboxylate on a surface of the positive electrode, reduce the formation of oxidation by-products, and inhibit the reduction and accumulation of the oxidation by-products on a surface of the negative electrode simultaneously. Therefore, the continuous increase of an impedance of the passivation film on the surfaces of the positive and negative electrodes may be avoided during cycling, and at the same time, the improvement effect of the carboxylate on a fast charging performance of the battery is ensured, thus inhibiting the capacity attenuation. The content of electrolyte salt and the content of carboxylate jointly affect viscosity and ion conductivity of the non-aqueous electrolyte. Under the control of comprehensive conditions, it is beneficial to form a dense passivation film on the surfaces of the positive and negative electrodes, and thus an internal resistance of the battery is effectively reduced and the fast charging performance and the cycle performance of the battery are improved.

From the test results of Comparative Examples 1 to 5, it may be seen that even if a value of a, a value of n and a value of m all meet parameter range limitation thereof. However, when a value of an/m is too large or too small, it results in the deterioration of the fast charging performance of the battery, indicating that there is an interaction among the mass percentage content a of the compound represented by the formula 1 in the non-aqueous electrolyte, the mass percentage content m of the carboxylate in the non-aqueous electrolyte and the mass percentage content n of the electrolyte salt in the non-aqueous electrolyte. The fast charging performance of the battery may be significantly improved only when the three are in a good balance state. At the same time, from the test results of Comparative Examples 6 to 13, it may be seen that when one of the value of a, the value of n and the value of m exceeds a defined range, even if the value of a, the value of n and the value of m satisfy the relational expression of $0.02 \leq an/m \leq 9$, the capacity retention rate and impedance growth rate of the battery under the condition of large current fast charging are poor, indicating that when the mass percentage content a of the compound represented by the formula 1 in the non-aqueous electrolyte, the mass percentage content m of the carboxylate in the non-aqueous electrolyte, and the mass percentage content n of the electrolyte salt in the non-aqueous electrolyte are too high or too low, the formation of the passivation film on the surfaces of the positive and the negative electrodes and the stability of the non-aqueous electrolyte under the condition of fast charging will be affected.

From the test results of Examples 1 to 30, it may be seen that lithium-ion batteries have a best comprehensive performance of the battery when the relational expression is $0.2 \leq an/m \leq 4$.

From the test results of Examples 8 to 12, it may be seen that in the lithium-ion battery provided in the present invention, with the increase of the content of the carboxylate in the solvent, the capacity retention rate and the impedance growth rate of the battery under the condition of fast charging increase first and then decrease, indicating that adding the carboxylate in an appropriate amount is beneficial to improve the conductivity of the non-aqueous electrolyte and is a prerequisite for improving the fast charging performance of the lithium-ion battery. However, the carboxylate is unstable. When the content of the carboxylate is too high or the compound represented by the formula 1 is not enough to inhibit the decomposition of the carboxylate, the carboxylate is prone to side reactions, which decomposes to increase a thickness of the passivation film on the surfaces of the positive and the negative electrodes, and reduce the conductivity of the non-aqueous electrolyte, thus affecting the capacity development and increasing the impedance of the lithium-ion battery.

From the test results of Examples 19 to 26, it may be seen that, with the increase of the content of the compound represented by the formula 1, the fast charging and cycle performance of the battery may be effectively improved only when the content of the carboxylate in the solvent is increased at the same time. When the content of the compound

represented by the formula 1 is high and the content of the carboxylate is insufficient, as shown in Example 25, the capacity retention rate and the impedance growth rate of the lithium-ion battery are slightly worse than those of Example 26, indicating that the passivation film formed by the compound represented by the formula 1 is relatively dense, and adequate carboxylate is needed to reduce the viscosity of the non-aqueous electrolyte, thus ensuring the infiltration of the non-aqueous electrolyte to the electrode material and improving the ionic conductivity.

(2) The test results obtained in Example 10 and Examples 31 to 35 are shown in Table 3.

Table 3

| Group | Capacity retention rate of 400 cycles at 45°C at 3C/3C (%) | Internal resistance growth rate of 400 cycles at 45°C at 3C/3C (%) |
|---|---|---|
| Example 10 | 89.1 | 40.1 |
| Example 31 | 88.5 | 40.5 |
| Example 32 | 88.3 | 41.0 |
| Example 33 | 88.6 | 40.1 |
| Example 34 | 88.2 | 41.2 |
| Example 35 | 88.1 | 41.3 |

From the test results of Example 10 and Examples 31-35, it may be seen that when compounds represented by different formulas 1 are used as the additives of the non-aqueous electrolyte, they also satisfy the limitation of the relational expression $0.02 \leq an/m \leq 9$, indicating that cyclic sulfur-containing groups commonly contained in the compounds represented by the different formulas 1 play a decisive role in the formation of passivation films on the surfaces of the positive and the negative electrodes. A passivation film rich in S element generated by decomposition has a good isolation effect on the carboxylate, thus avoiding the continuous decomposition of the carboxylate on the surfaces of positive and negative electrode materials. The relational expression defined in the present invention is universal for the compounds represented by the different formula 1, and has an improvement effect on the fast charging and cycle performance of lithium-ion battery.

(3) Test results obtained in Example 10 and Examples 36 to 38 are shown in Table 4.

Table 4

| Group | Capacity retention rate of 400 cycles at 45°C at 3C/3C (%) | Internal resistance growth rate of 400 cycles at 45°C at 3C/3C (%) |
|---|---|---|
| Example 10 | 89.1 | 40.1 |
| Example 36 | 88.9 | 42.1 |
| Example 37 | 88.4 | 44.1 |
| Example 38 | 88.7 | 42.5 |

From the test results of Example 10 and Examples 36 to 38, it may be seen that, when different carboxylates are used as the non-aqueous organic solvents, they also satisfy the limitation of the relational expression $0.02 \leq an/m \leq 9$, indicating that different carboxylates have the functions of reducing the viscosity of the non-aqueous electrolyte and improving the conductivity, and also have a good synergistic effect with the compound represented by the formula 1, and may synergistically improve the capacity retention rate and reduce the impedance growth rate of the battery under the condition of fast charging.

(4) Test results obtained in Example 10 and Examples 39 to 43 are shown in Table 5.

Table 5

| Group | Capacity retention rate of 400 cycles at 45°C at 3C/3C (%) | Internal resistance growth rate of 400 cycles at 45°C at 3C/3C (%) |
|---|---|---|
| Example 10 | 89.1 | 40.1 |
| Example 39 | 88.7 | 37.1 |
| Example 40 | 90.0 | 37.4 |

(continued)

| Group | Capacity retention rate of 400 cycles at 45°C at 3C/3C (%) | Internal resistance growth rate of 400 cycles at 45°C at 3C/3C (%) |
|---|---|---|
| Example 41 | 89.0 | 37.4 |
| Example 42 | 88.4 | 38.4 |
| Example 43 | 91.2 | 39.5 |

From the test results of Example 10 and Examples 39 to 43, it may be seen that on the basis of the battery provided in the present invention, 1,3-propane sultone (PS), ethylene sulfate (DTD), tripropargyl phosphate, succinonitrile or LiODFB are added as the auxiliary additive, so that the capacity retention performance of the battery may be further improved, and the impedance growth rate of the battery may be reduced. It is speculated that due to the fact that a certain common decomposition reaction exists between the compound represented by the formula 1 and added 1,3-propane sultone (PS), ethylene sulfate (DTD), tripropargyl phosphate, succinonitrile or LiODFB, which may jointly participate in the formation of passivation film on an electrode surface. An obtained passivation film may improve the stability of the non-aqueous electrolyte and maintain the stability of the cycle of the battery and high current resistance.

The above descriptions are only preferred embodiments of the present invention, and are not intended to limit the present invention.

## Claims

1. A non-aqueous electrolyte, comprising an electrolyte salt, a non-aqueous organic solvent, and an additive, wherein the non-aqueous organic solvent comprises a carboxylate, and the additive comprises a compound represented by formula 1:

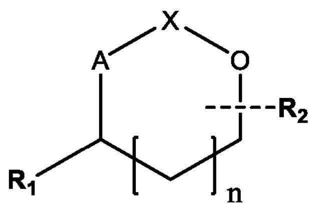

Formula 1

where n is 0 or 1, A is selected from C or O, X is selected from

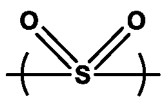

or

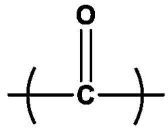

,

$R_1$ and $R_2$ are each independently selected from H,

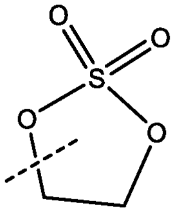

,

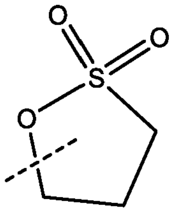 , 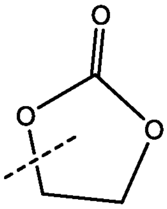 , 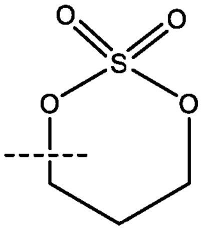 , or 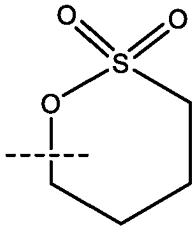 ,

$R_1$ and $R_2$ are not selected from H simultaneously, and X, $R_1$ and $R_2$ at least contain one sulfur atom; wherein the non-aqueous electrolyte satisfies following conditions:

$0.02 \le an/m \le 9$, $0.01\% \le a \le 5\%$, $5\% \le m \le 70\%$, and $8\% \le n \le 25\%$;
where a is a mass percentage content of the compound represented by the formula 1 in the non-aqueous electrolyte, in %;
m is a mass percentage content of the carboxylate in the non-aqueous electrolyte, in %; and
n is a mass percentage content of the electrolyte salt in the non-aqueous electrolyte, in %;
wherein a conductivity of the non-aqueous electrolyte at 25°C is greater than or equal to 5mS/cm.

**2.** The non-aqueous electrolyte of claim 1, wherein the non-aqueous electrolyte satisfies a following condition:

$$0.2 \le an/m \le 4.$$

**3.** The non-aqueous electrolyte of claim 1, wherein the mass percentage content a of the compound represented by the formula 1 in the non-aqueous electrolyte is 0.05% to 3%.

**4.** The non-aqueous electrolyte of claim 1, wherein the mass percentage content m of the carboxylate in the non-aqueous electrolyte is 10% to 60%.

**5.** The non-aqueous electrolyte of claim 1, wherein the mass percentage content n of the electrolyte salt in the non-aqueous electrolyte is 10% to 20%.

**6.** The non-aqueous electrolyte of claim 5, wherein the electrolyte salt is selected from one or more of: $LiPF_6$, $LiPO_2F_2$, $LiBF_4$, $LiClO_4$, $LiCF_3SO_3$, $LiN(SO_2CF_3)_2$ and $LiN(SO_2F)_2$.

**7.** The non-aqueous electrolyte of claim 1, wherein the compound represented by the formula 1 is selected from one or more of following compounds 1-22:

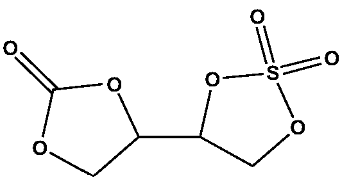

Compound 1

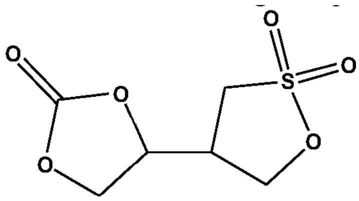

Compound 2

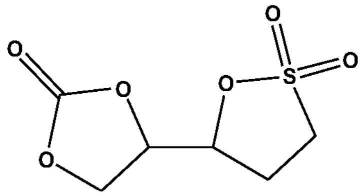

Compound 3

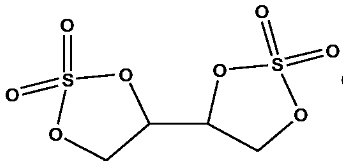

Compound 4

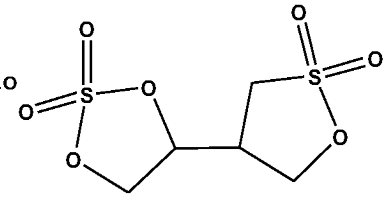

Compound 5

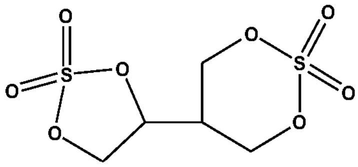

Compound 6

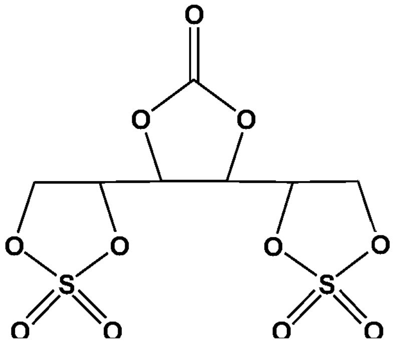

Compound 7

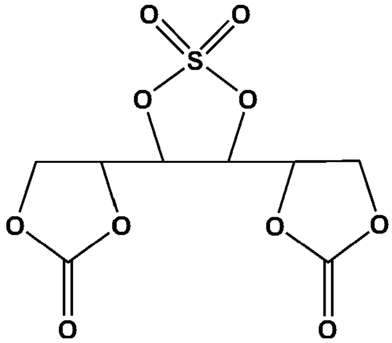

Compound 8

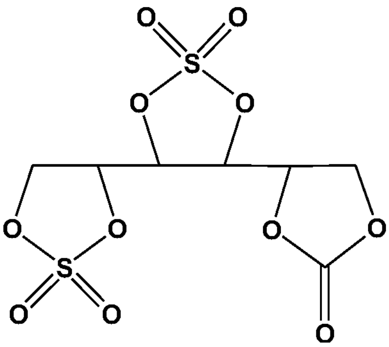

Compound 9

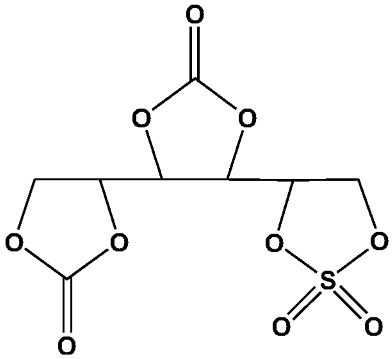

Compound 10

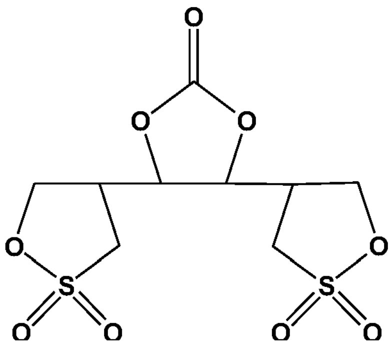

Compound 11

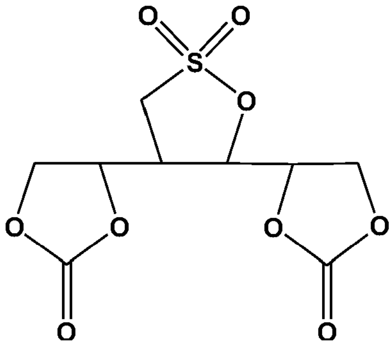

Compound 12

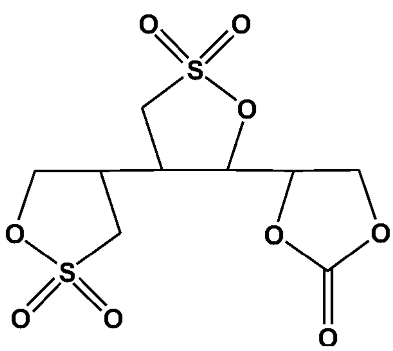

Compound 13

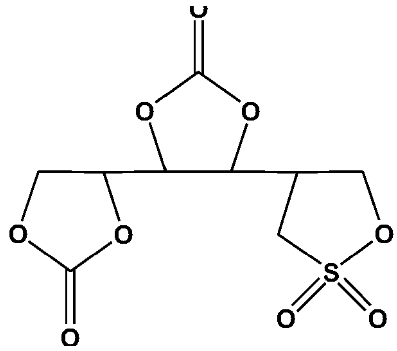

Compound 14

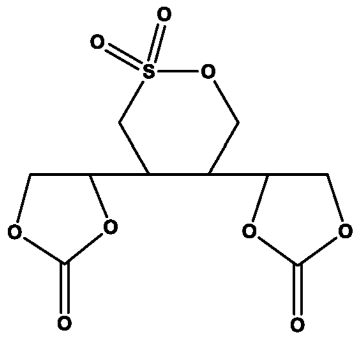

Compound 15

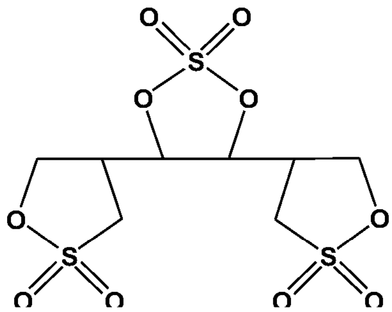

Compound 16

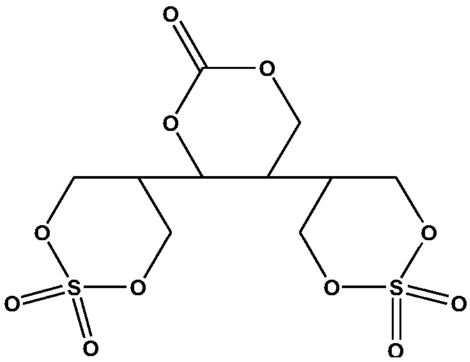

Compound 17

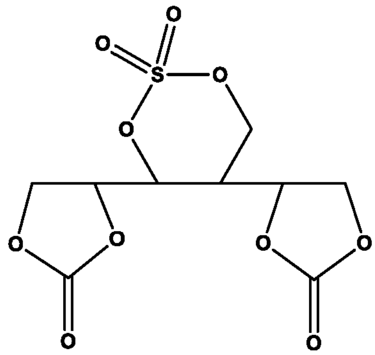

Compound 18

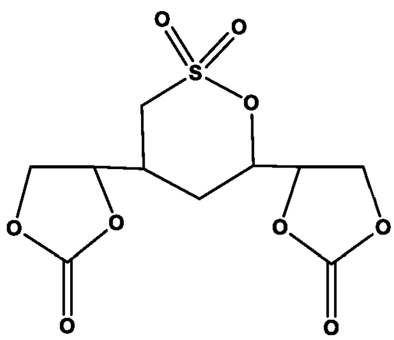

Compound 19

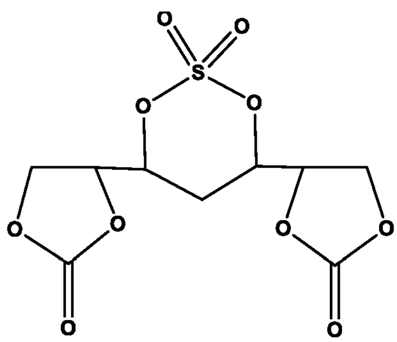

Compound 20

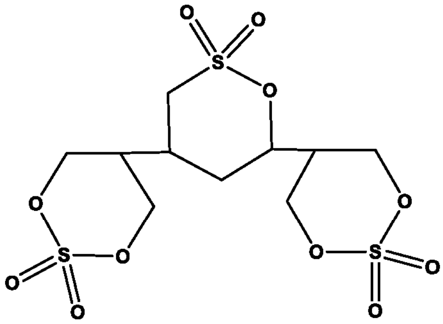

Compound 21

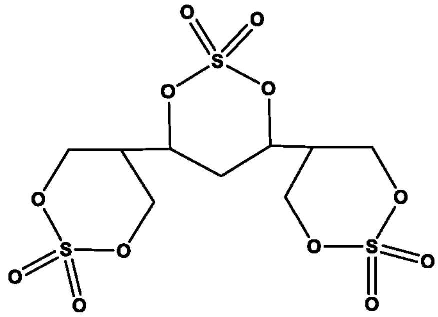

Compound 22

8. The non-aqueous electrolyte of claim 1, wherein the carboxylate comprises one or more of: methyl acetate, ethyl acetate, propyl acetate, methyl propionate, ethyl propionate, propyl propionate, 2,2-difluoroethyl acetate, methyl butyrate, methyl isobutyrate, methyl trimethylacetate and ethyl trimethylacetate.

9. The non-aqueous electrolyte of claim 1, further comprising an auxiliary additive, wherein the auxiliary additive comprises at least one of: a cyclic sulfate compound, a sultone compound, a cyclic carbonate compound, an unsaturated phosphate compound, a nitrile compound and a lithium oxalate borate.

10. The non-aqueous electrolyte of claim 9, wherein the auxiliary additive is added in an amount of 0.01% to 30% based on a total mass of the non-aqueous electrolyte as 100%.

11. The non-aqueous electrolyte of claim 9, wherein the cyclic sulfate compound is selected from at least one of: ethylene sulfate, propene sulfate or methyl ethylene sulfate;

wherein the sultone compound is selected from at least one of: 1,3-propane sultone, 1,4-butane sultone or 1,3-propene sultone; or
wherein the cyclic carbonate compound is selected from at least one of: vinylene carbonate, vinyl ethylene carbonate, fluoroethylene carbonate or a compound represented by formula 2,

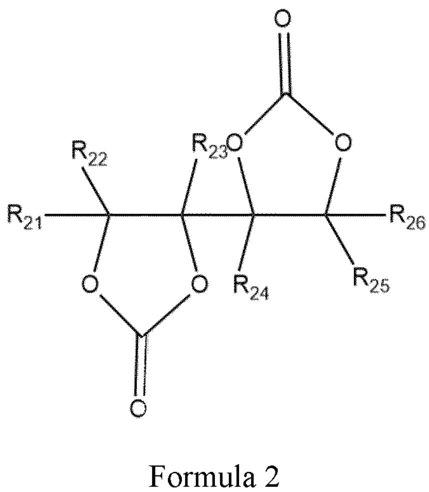

Formula 2

in the formula 2, $R_{21}$, $R_{22}$, $R_{23}$, $R_{24}$, $R_{25}$ and $R_{26}$ are each independently selected from one of: a hydrogen atom, a halogen atom or a C1-C5 group.

12. The non-aqueous electrolyte of claim 9, wherein the unsaturated phosphate compound is selected from at least one of compounds represented by formula 3:

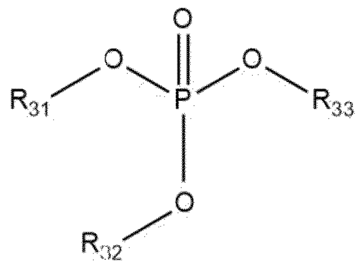

Formula 3

in the formula 3, $R_{31}$, $R_{32}$ and $R_{33}$ are each independently selected from a C1-C5 saturated hydrocarbon group, an unsaturated hydrocarbon group, a halogenated hydrocarbon group and -$Si(C_mH_{2m+1})_3$, where m is a natural number of 1 to 3, and at least one of $R_{31}$, $R_{32}$ and $R_{33}$ is an unsaturated hydrocarbon group;
wherein the nitrile compound is selected from one or more of: succinonitrile, glutaronitrile, ethylene glycol bis(propionitrile)ether, hexane trinitrile, adiponitrile, pimelonitrile, suberonitrile, azelanitrile and sebaconitrile; or
wherein the lithium oxalate borate is selected from one or more of: $Li[B(C_2O_4)_2]$ and $Li[B(C_2O_4)F_2]$.

**13.** The non-aqueous electrolyte of claim 1, wherein the non-aqueous organic solvent further comprises one or more of: cyclic carbonates, linear carbonates and ethers.

**14.** The non-aqueous electrolyte of claim 13, wherein

the cyclic carbonates comprise one or more of: vinylene carbonate, propene carbonate and ethylene carbonate;
the linear carbonates comprise one or more of: dimethyl carbonate, diethyl carbonate and ethyl methyl carbonate; or
the ethers comprise one or more of: glycol dimethyl ether, 1,3-dioxolane and 1,1,2,2-tetrafluoroethyl-2,2,3,3-tetrafluoropropylether.

**15.** A secondary battery, comprising a positive electrode, a negative electrode, and a non-aqueous electrolyte according to any one of claims 1 to 14, wherein the positive electrode comprises a positive electrode material layer, and a compaction density of the positive electrode material layer is 3.5g/$cm^3$ or more, and the negative electrode comprises a negative electrode material layer, and a compaction density of the negative electrode material layer is 1.5g/$cm^3$ or more.

**Patentansprüche**

**1.** Nicht-wässriger Elektrolyt, umfassend ein Elektrolytsalz, ein nicht-wässriges organisches Lösungsmittel und einen Zusatzstoff, worin das nicht-wässrige organische Lösungsmittel ein Carboxylat umfasst, und der Zusatzstoff eine Verbindung umfasst, die von Formel 1 dargestellt ist:

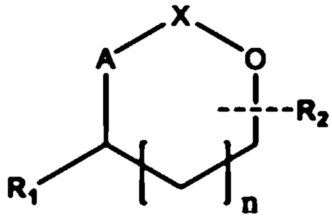

Formel 1

wo n 0 oder 1 ist, A aus C oder O ausgewählt ist, X aus

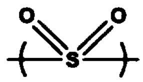

oder

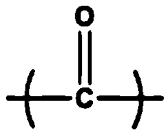

ausgewählt ist, $R_1$ und $R_2$ jeder unabhängig aus H,

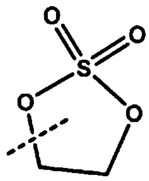,

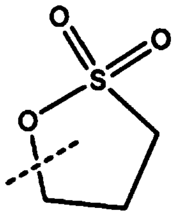, 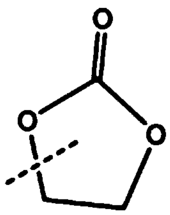, 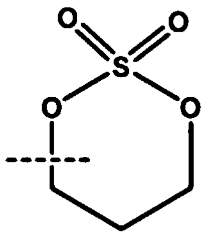, oder 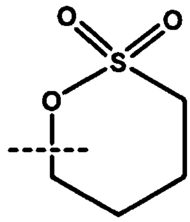.

ausgewählt sind, $R_1$ und $R_2$ nicht aus H gleichzeitig ausgewählt sind, und X, $R_1$ und $R_2$ zumindest ein Schwefelatom enthalten;
worin der nicht-wässrige Elektrolyt die folgenden Bedingungen erfüllt:

$0.02 \leq an/m \leq 9$, $0.01\% \leq a \leq 5\%$, $5\% \leq m \leq 70\%$ und $8\% \leq n \leq 25\%$;
wo a ein Massenprozentgehalt der Verbindung, die von Formel 1 dargestellt ist, im nicht-wässrigen Elektrolyt, in % ist;
m ein Massenprozentgehalt des Carboxylats im nicht-wässrigen Elektrolyt, in % ist; und
n ein Massenprozentgehalt des Elektrolytsalzes im nicht-wässrigen Elektrolyt, in % ist;
worin eine Leitfähigkeit des nicht-wässrigen Elektrolyts bei 25°C größer als oder gleich 5mS/cm ist.

2. Nicht-wässriger Elektrolyt nach Anspruch 1, worin der nicht-wässrige Elektrolyt eine folgende Bedingung erfüllt:

$$0.2 < an/m \leq 4.$$

3. Nicht-wässriger Elektrolyt nach Anspruch 1, worin der Massenprozentgehalt a der Verbindung, die von Formel 1 dargestellt ist, im nicht-wässrigen Elektrolyt 0.05% bis 3% ist.

4. Nicht-wässriger Elektrolyt nach Anspruch 1, worin der Massenprozentgehalt m des Carboxylats im nicht-wässrigen Elektrolyt 10% bis 60% ist.

5. Nicht-wässriger Elektrolyt nach Anspruch 1, worin der Massenprozentgehalt n des Elektrolytsalzes im nicht-wässrigen Elektrolyt 10% bis 20% ist.

6. Nicht-wässriger Elektrolyt nach Anspruch 5, worin das Elektrolytsalz aus einem oder mehreren von $LiPF_6$, $LiPO_2F_2$, $LiBF_4$, $LiClO_4$, $LiCF_3SO_3$, $LiN(SO_2CF_3)_2$ und $LiN(SO_2F)_2$ ausgewählt ist.

7. Nicht-wässriger Elektrolyt nach Anspruch 1, worin die Verbindung, die von Formel 1 dargestellt ist, aus einem oder mehreren von folgenden Verbindungen 1-22 ausgewählt ist:

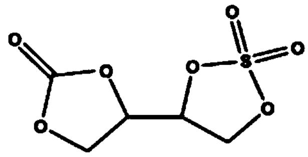
Verbindung 1

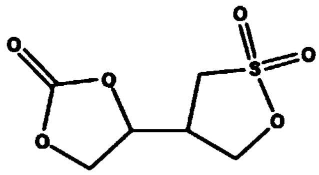
Verbindung 2

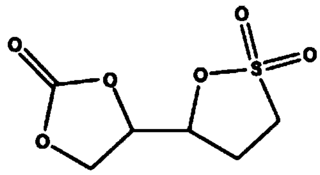
Verbindung 3

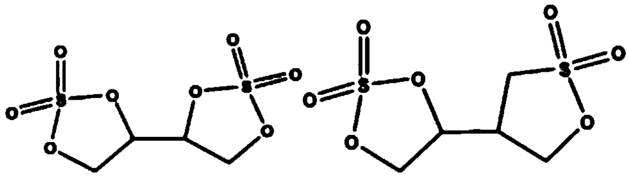
Verbindung 4

Verbindung 5

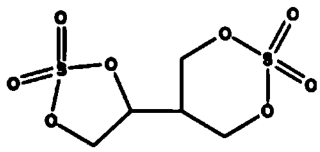
Verbindung 6

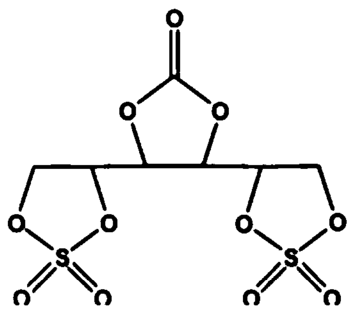
Verbindung 7

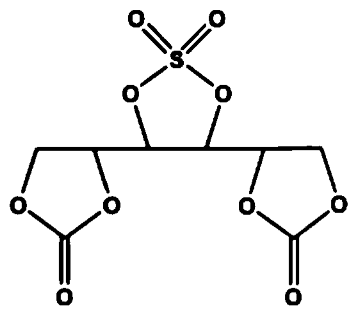
Verbindung 8

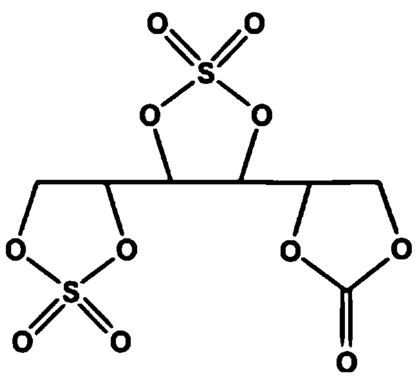
Verbindung 9

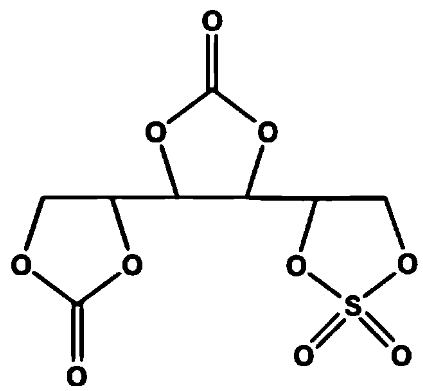
Verbindung 10

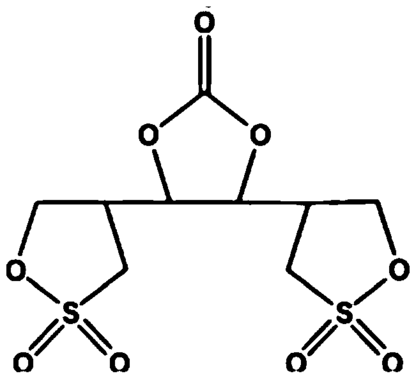

Verbindung 11

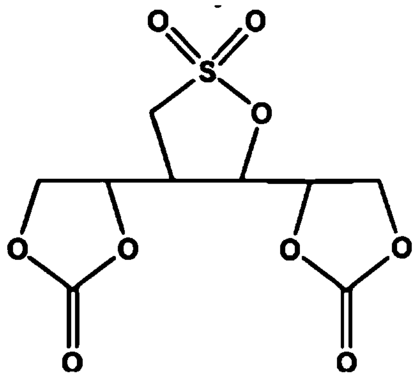

Verbindung 12

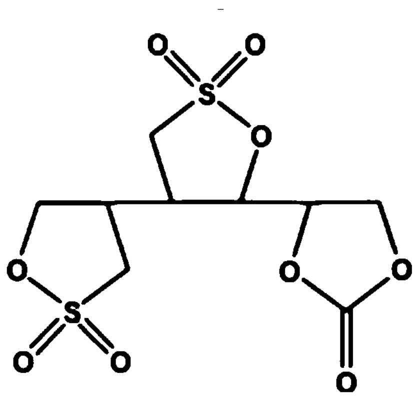

Verbindung 13

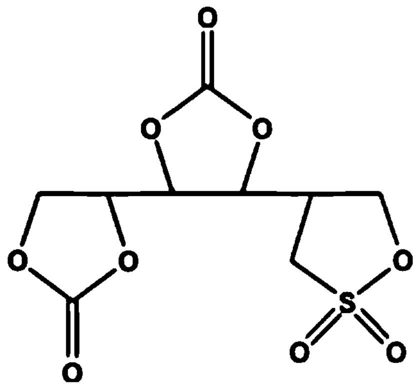

Verbindung 14

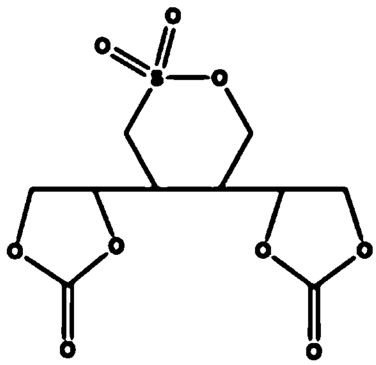

Verbindung 15

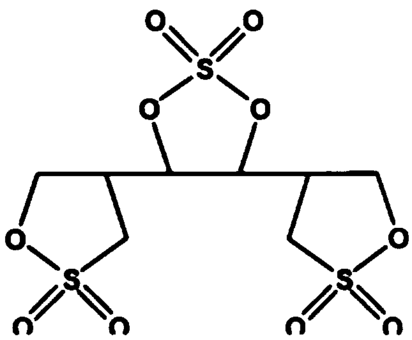

Verbindung 16

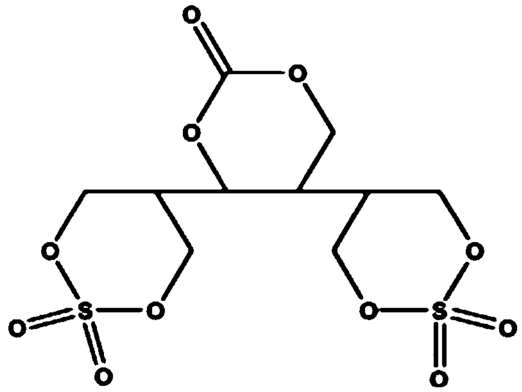

Verbindung 17

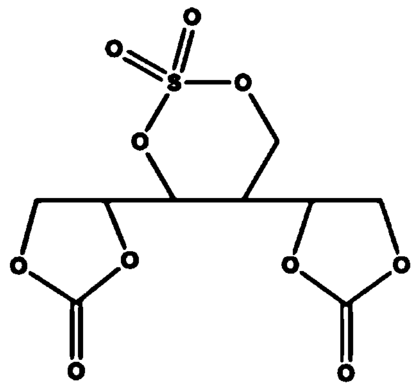

Verbindung 18

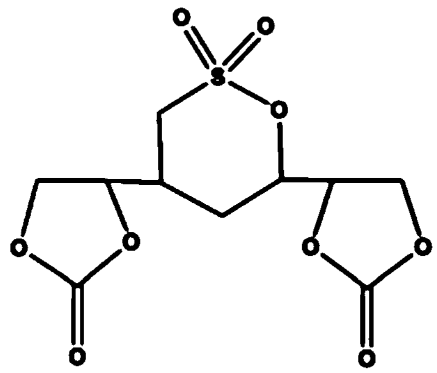

Verbindung 19

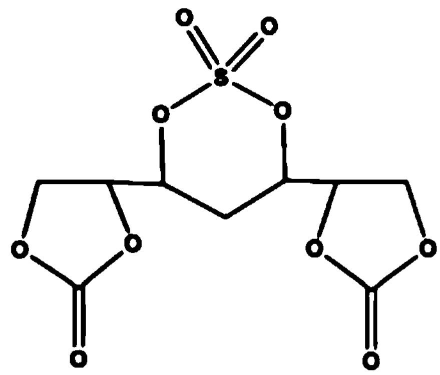

Verbindung 20

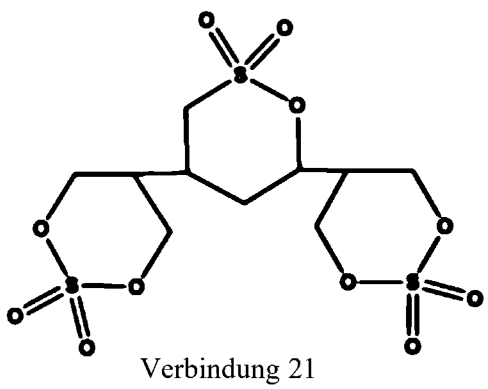

Verbindung 21

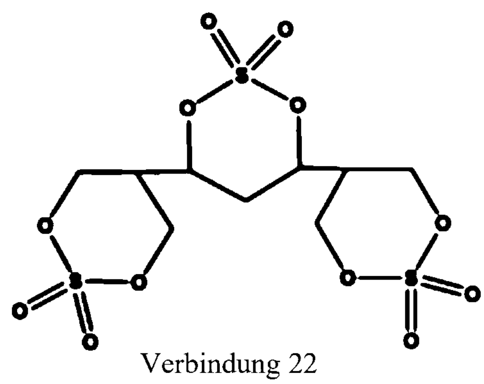

Verbindung 22

**8.** Nicht-wässriger Elektrolyt nach Anspruch 1, worin das Carboxylat eines oder mehrere von folgenden umfasst: Methylacetat, Ethylacetat, Propylacetat, Methylpropionat, Ethylpropionat, Propylpropionat, 2,2-Difluoroethylacetat, Methylbutyrat, Methylisobutyrat, Methyltrimethylacetat und Ethyltrimethylacetat.

**9.** Nicht-wässriger Elektrolyt nach Anspruch 1, ferner umfassend ein Hilfszusatzstoff, worin der Hilfszusatzstoff zumindest eine von folgenden umfasst: einer cyclischen Sulfatverbindung, einer Sultonverbindung, einer cyclischen Carbonatverbindung, einer ungesättigten Phosphatverbindung, einer Nitrilverbindung und einem Lithiumoxalatborat.

**10.** Nicht-wässriger Elektrolyt nach Anspruch 9, worin der Hilfszusatzstoff in einer Menge von 0.01% bis 30% aufgrund einer Gesamtmasse des nicht-wässrigen Elektrolyts als 100% zugesetzt wird.

**11.** Nicht-wässriger Elektrolyt nach Anspruch 9, worin die cyclische Sulfatverbindung aus zumindest einem von Ethylensulfat, Propensulfat oder Methylethylensulfat ausgewählt ist;

worin die Sultonverbindung aus zumindest einem von 1,3-Propansulton, 1,4-Butansulton oder 1,3-Propensulton ausgewählt ist; oder

worin die cyclische Carbonatverbindung aus zumindest einem von Vinylencarbonat, Vinylethylencarbonat, Fluoroethylencarbonat oder einer Verbindung, die von Formel 2 dargestellt ist, ausgewählt ist:

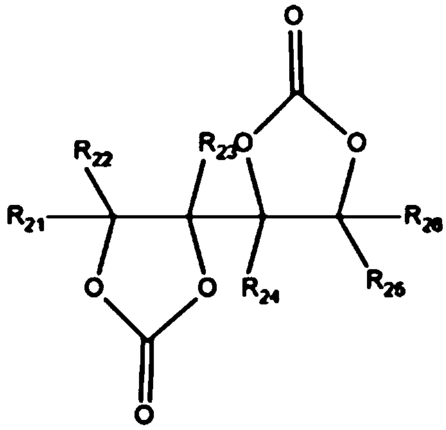

Formel 2

in der Formel 2, $R_{21}$, $R_{22}$, $R_{23}$, $R_{24}$, $R_{25}$ und $R_{26}$ jeder unabhängig aus einem von einem Wasserstoffatom, einem Halogenatom oder einer C1-C5 Gruppe ausgewählt sind.

**12.** Nicht-wässriger Elektrolyt nach Anspruch 9, worin die ungesättigte Phosphatverbindung aus zumindest einer von den Verbindungen, die von Formel 3 dargestellt sind, ausgewählt ist:

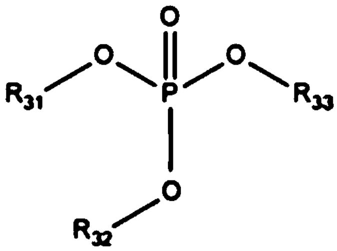

Formel 3

in der Formel 3, $R_{31}$, $R_{32}$ und $R_{33}$ jeder unabhängig aus einer C1-C5-gesättigten Kohlenwasserstoffgruppe, einer ungesättigten Kohlenwasserstoffgruppe, einer halogenierten Kohlenwasserstoffgruppe und $-Si(C_mH_{2m+1})_3$ ausgewählt sind, wo m eine natürliche Zahl von 1 bis 3 ist und zumindest einer von $R_{31}$, $R_{32}$ und $R_{33}$ eine ungesättigte Kohlenwasserstoffgruppe ist;

worin die Nitrilverbindung aus einem oder mehreren von Succinonitril, Glutaronitril, Ethylenglycol-Bis(propionitril)ether, Hexantrinitril, Adiponitril, Pimelonitril, Suberonitril, Azelanitril und Sebaconitril ausgewählt ist; oder

worin das Lithiumoxalatborat aus einem oder mehreren von $Li[B(C_2O_4)_2]$ und $Li[B(C_2O_4)F_2]$ ausgewählt ist.

**13.** Nicht-wässriger Elektrolyt nach Anspruch 1, worin das nicht-wässrige organische Lösungsmittel ferner eines oder mehrere von cyclischen Carbonaten, linearen Carbonaten und Ethern umfasst.

**14.** Nicht-wässriger Elektrolyt nach Anspruch 13, worin

die cyclischen Carbonate eines oder mehrere von Vinylencarbonat, Propencarbonat und Ethylencarbonat umfassen;

die linearen Carbonate eines oder mehrere von Dimethylcarbonat, Diethylcarbonat und Ethylmethylcarbonat umfassen; oder

die Ether einen oder mehreren von Glycoldimethylether, 1,3-Dioxolan und 1,1,2,2-Tetrafluoroethyl-2,2,3,3-Tetrafluoropropylether umfassen.

**15.** Sekundärbatterie, umfassend eine positive Elektrode, eine negative Elektrode und einen nicht-wässrigen Elektrolyt nach einem der Ansprüche 1 bis 14, worin die positive Elektrode eine positive Elektrodenmaterialschicht umfasst, und eine Verdichtungsdichte der positiven Elektrodenmaterialschicht 3.5g/$cm^3$ oder mehr ist, und die negative Elektrode eine negative Elektrodenmaterialschicht umfasst, und eine Verdichtungsdichte der negativen Elektrodenmaterialschicht 1.5g/$cm^3$ oder mehr ist.

## Revendications

**1.** Électrolyte non aqueux, comprenant un sel d'électrolyte, un solvant organique non aqueux et un additif, dans lequel le solvant organique non aqueux comprend un carboxylate, et l'additif comprend un composé représenté par la formule 1 :

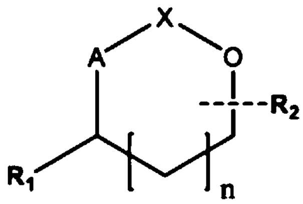

Formule 1

dans lequel n est 0 ou 1, A est sélectionné parmi C ou O, X est sélectionné parmi

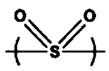 ou 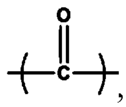,

$R_1$ et $R_2$ sont chacun indépendamment sélectionnés parmi H,

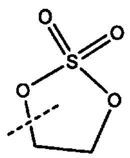, 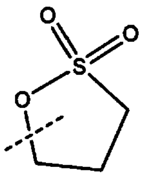, 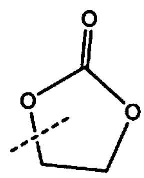, 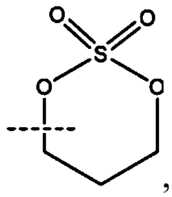,

ou

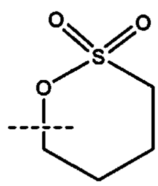,

$R_1$ et $R_2$ ne sont pas sélectionnés parmi H simultanément, et X, $R_1$ et $R_2$ contiennent au moins un atome de soufre ;
dans lequel l'électrolyte non aqueux satisfait aux conditions suivantes :

$0.02 \leq an/m \leq 9$, $0.01\% \leq a \leq 5\%$, $5\% \leq m \leq 70\%$ et $8\% \leq n \leq 25\%$ ;
dans lequel a est une teneur en pourcentage massique du composé représenté par la formule 1 dans l'électrolyte non aqueux, en % ;
m est une teneur en pourcentage massique du carboxylate dans l'électrolyte non aqueux, en % ; et

n est une teneur en pourcentage massique du sel d'électrolyte dans l'électrolyte non aqueux, en % ; dans lequel une conductivité de l'électrolyte non aqueux à 25°C est supérieure ou égale à 5mS/cm.

2. Électrolyte non aqueux selon la revendication 1, dans lequel l'électrolyte non aqueux satisfait à une condition suivante :

$$0.2 \leq an/m \leq 4.$$

3. Électrolyte non aqueux selon la revendication 1, dans lequel la teneur en pourcentage massique a du composé représenté par la formule 1 dans l'électrolyte non aqueux est de 0.05% à 3%.

4. Électrolyte non aqueux selon la revendication 1, dans lequel la teneur en pourcentage massique m du carboxylate dans l'électrolyte non aqueux est de 10% à 60%.

5. Électrolyte non aqueux selon la revendication 1, dans lequel la teneur en pourcentage massique n du sel d'électrolyte dans l'électrolyte non aqueux est de 10% à 20%.

6. Électrolyte non aqueux selon la revendication 5, dans lequel le sel d'électrolyte est sélectionné parmi un ou plusieurs des éléments suivants : $LiPF_6$, $LiPO_2F_2$, $LiBF_4$, $LiClO_4$, $LiCF_3SO_3$, $LiN(SO_2CF_3)_2$ et $LiN(SO_2F)_2$.

7. Électrolyte non aqueux selon la revendication 1, dans lequel le composé représenté par la formule 1 est sélectionné parmi un ou plusieurs des composés 1-22 suivants :

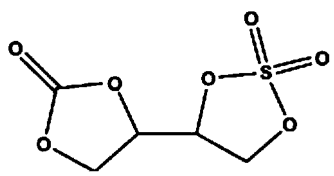

Composé 1

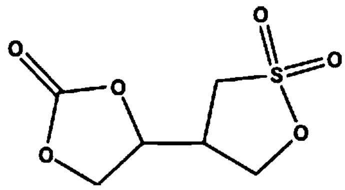

Composé 2

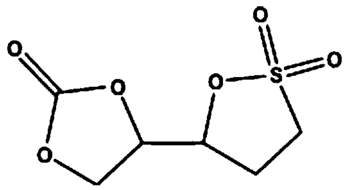

Composé 3

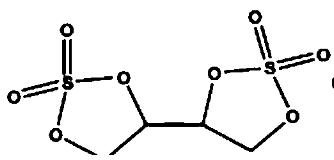

Composé 4

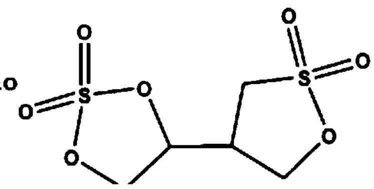

Composé 5

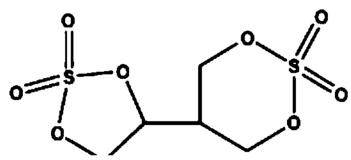

Composé 6

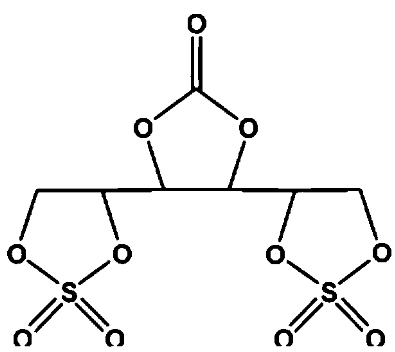

Composé 7

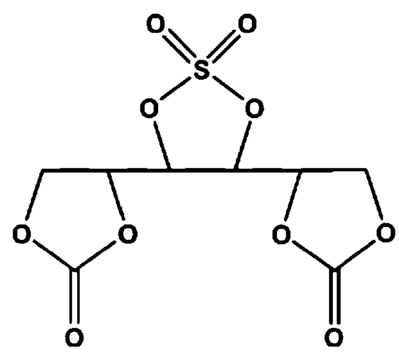

Composé 8

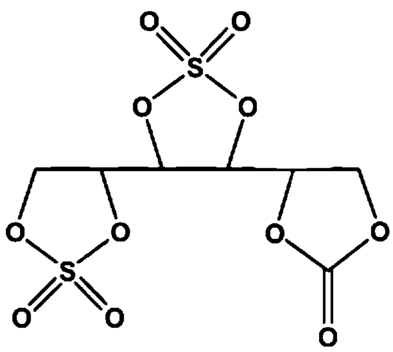

Composé 9

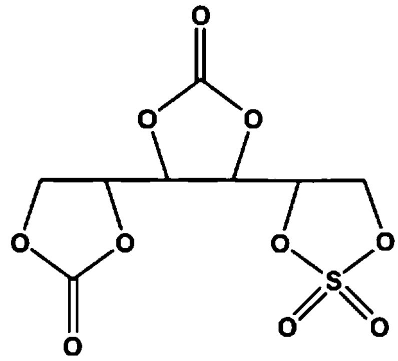

Composé 10

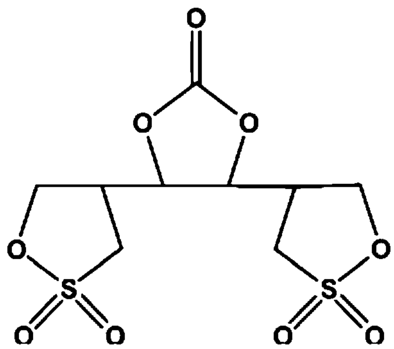

Composé 11

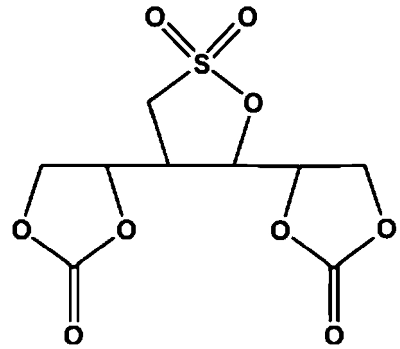

Composé 12

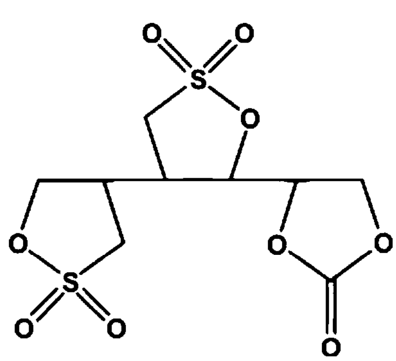

Composé 13

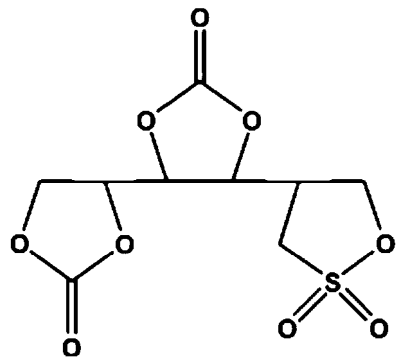

Composé 14

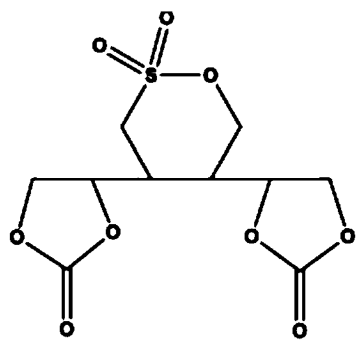

Composé 15

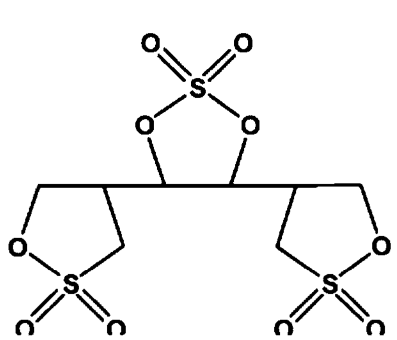

Composé 16

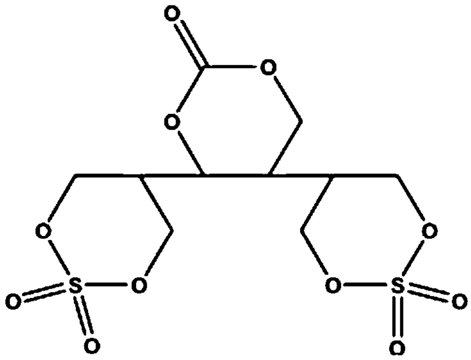

Composé 17

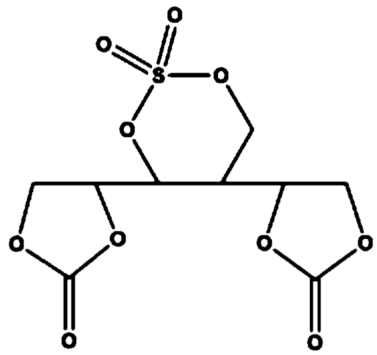

Composé 18

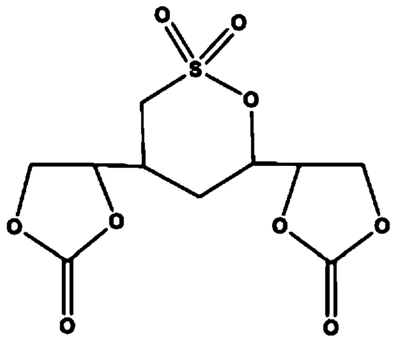

Composé 19

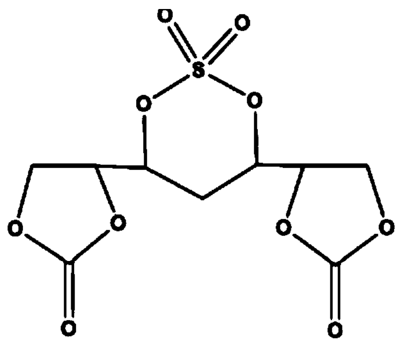

Composé 20

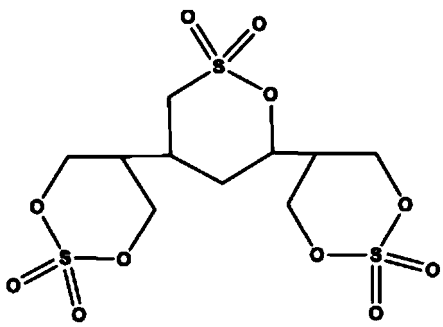

Composé 21

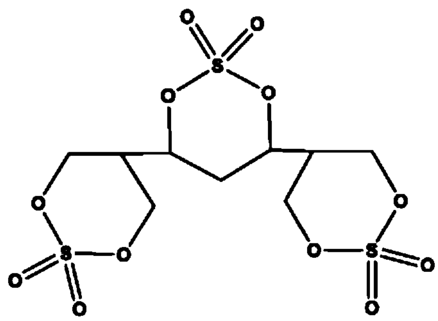

Composé 22

**8.** Électrolyte non aqueux selon la revendication 1, dans lequel le carboxylate comprend un ou plusieurs des éléments parmi : acétate de méthyle, acétate d'éthyle, acétate de propyle, propionate de méthyle, propionate d'éthyle, propionate de propyle, acétate de 2,2-difluoroéthyle, butyrate de méthyle, isobutyrate de méthyle, triméthylacétate de méthyle et triméthylacétate d'éthyle.

**9.** Électrolyte non aqueux selon la revendication 1, comprenant en outre un additif auxiliaire, dans lequel l'additif auxiliaire comprend au moins un élément parmi : un composé sulfate cyclique, un composé sultone, un composé carbonate cyclique, un composé phosphate insaturé, un composé nitrile et un oxalate borate de lithium.

**10.** Électrolyte non aqueux selon la revendication 9, dans lequel l'additif auxiliaire est ajouté en une quantité de 0.01% à 30% par rapport à une masse totale de l'électrolyte non aqueux de 100%.

**11.** Électrolyte non aqueux selon la revendication 9, dans lequel le composé sulfate cyclique est au moins un élément sélectionné parmi : sulfate d'éthylène, sulfate de propène ou sulfate de méthyle et d'éthylène ;

dans lequel le composé sultone est au moins un élément sélectionné parmi : propane-1,3-sultone, butane-1,4-sultone ou propène-1,3-sultone ; ou
dans lequel le composé carbonate cyclique est au moins un élément sélectionné parmi : carbonate de vinylène, carbonate d'éthylène vinylique, carbonate de fluoroéthylène ou un composé représenté par la formule 2,

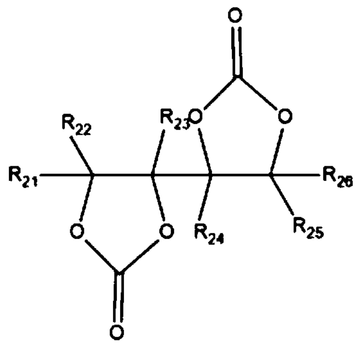

Formule 2

dans la formule 2, $R_{21}$, $R_{22}$, $R_{23}$, $R_{24}$, $R_{25}$ et $R_{26}$ sont chacun indépendamment sélectionnés parmi : un atome d'hydrogène, un atome d'halogène ou un groupe en C1-C5.

**12.** Électrolyte non aqueux selon la revendication 9, dans lequel le composé phosphate insaturé est sélectionné parmi au moins l'un des composés représentés par la formule 3 :

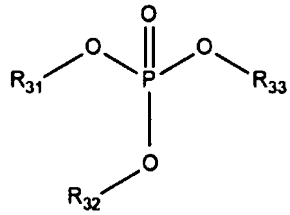

Formule 3

dans la formule 3, $R_{31}$, $R_{32}$ et $R_{33}$ sont chacun indépendamment sélectionnés parmi un groupe hydrocarboné saturé en C1-C5, un groupe hydrocarboné insaturé, un groupe hydrocarboné halogéné et $-Si(C_mH_{2m+1})_3$, dans lequel m est un nombre naturel de 1 à 3, et au moins l'un de $R_{31}$, $R_{32}$ et $R_{33}$ est un groupe hydrocarboné insaturé ;
dans lequel le composé nitrile est un ou plusieurs éléments sélectionnés parmi : succinonitrile, glutaronitrile, éther bis(propionitrilique) d'éthylène glycol, hexane trinitrile, adiponitrile, pimélonitrile, subéronitrile, azélanitrile et sébaconitrile ; ou
dans lequel l'oxalate borate de lithium est un ou plusieurs éléments sélectionnés parmi : $Li[B(C_2O_4)_2]$ et $Li[B(C_2O_4)F_2]$.

**13.** Électrolyte non aqueux selon la revendication 1, dans lequel le solvant organique non aqueux comprend en outre un ou plusieurs éléments parmi : carbonates cycliques, carbonates linéaires et éthers.

**14.** Électrolyte non aqueux selon la revendication 13, dans lequel

les carbonates cycliques comprennent un ou plusieurs éléments parmi : carbonate de vinylène, carbonate de propène et carbonate d'éthylène ;
les carbonates linéaires comprennent un ou plusieurs éléments parmi : carbonate de diméthyle, carbonate de diéthyle et carbonate d'éthyle et de méthyle ; ou
les éthers comprennent un ou plusieurs éléments parmi : éther diméthylique de glycol, 1,3-dioxolane et 1,1,2,2-tétrafluoroéthyl-2,2,3,3-tétrafluoropropyléther.

**15.** Batterie secondaire comprenant une électrode positive, une électrode négative et un électrolyte non aqueux selon l'une quelconque des revendications 1 à 14, dans laquelle l'électrode positive comprend une couche de matériau

d’électrode positive et une densité de compactage de la couche de matériau d’électrode positive est supérieure ou égale à 3.5g/$cm^3$, et l’électrode négative comprend une couche de matériau d’électrode négative et une densité de compactage de la couche de matériau d’électrode négative est supérieure ou égale à 1.5g/$cm^3$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 112349961 A